(19) **Europäisches Patentamt / European Patent Office / Office européen des brevets**

(11) **EP 4 159 840 B1**

(12) **EUROPEAN PATENT SPECIFICATION**

(45) Date of publication and mention
of the grant of the patent:
**08.07.2026 Bulletin 2026/28**

(21) Application number: **21813992.1**

(22) Date of filing: **28.05.2021**

(51) International Patent Classification (IPC):
**C12N 1/20** (2026.01)  **A01N 63/22** (2020.01)
**A01P 3/00** (2006.01)  **C12R 1/07** (2006.01)

(52) Cooperative Patent Classification (CPC):
**A01N 63/22; A01P 3/00; C12N 1/205;**
C12R 2001/07

(86) International application number:
**PCT/JP2021/020546**

(87) International publication number:
**WO 2021/241759 (02.12.2021 Gazette 2021/48)**

(54) **BACTERIAL STRAIN BELONGING TO BACILLUS GENUS, AND MICROBIOLOGICAL CONTROL AGENT USING SAID BACTERIAL STRAIN**

BAKTERIENSTAMM DER GATTUNG BACILLUS UND MIKROBIOLOGISCHES BEKÄMPFUNGSMITTEL MIT DIESEM BAKTERIENSTAMM

SOUCHE BACTÉRIENNE APPARTENANT AU GENRE BACILLUS, ET PESTICIDE MICROBIEN METTANT EN OEUVRE CETTE SOUCHE BACTÉRIENNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.05.2020 JP 2020094859**

(43) Date of publication of application:
**05.04.2023 Bulletin 2023/14**

(73) Proprietors:
• **National University Corporation Tokai National Higher Education and Research System**
Nagoya-shi, Aichi 464-8601 (JP)
• **Kumiai Chemical Industry Co., Ltd.**
Taito-ku
Tokyo 110-8782 (JP)

(72) Inventors:
• **SHIMIZU, Masafumi**
Nagoya-shi, Aichi 464-8601 (JP)
• **KANEKO, Isao**
Tokyo 110-8782 (JP)
• **OHTAKA, Nobuaki**
Tokyo 110-8782 (JP)
• **MARUYAMA, Nozomu**
Tokyo 110-8782 (JP)

(74) Representative: **Vossius & Partner Patentanwälte Rechtsanwälte mbB**
**Siebertstraße 3**
**81675 München (DE)**

(56) References cited:
**WO-A1-2019/222253    JP-A- 2005 272 434**
**JP-A- 2007 055 982**

• **LIU KE ET AL:** "Induction of systemic resistance in Chinese cabbage against black rot by plant growth-promoting rhizobacteria", BIOLOGICAL CONTROL, SAN DIEGO, CA, US, vol. 99, 13 April 2016 (2016-04-13), pages 8 - 13, XP029594376, ISSN: 1049-9644, DOI: 10.1016/ J.BIOCONTROL.2016.04.007
• **TSUDA KAZUHISA ET AL:** "Biological control of bacterial soft rot in Chinese cabbage byLactobacillus plantarumstrain BY under field conditions", BIOLOGICAL CONTROL, SAN DIEGO, CA, US, vol. 100, 20 May 2016 (2016-05-20), pages 63 - 69, XP029631001, ISSN: 1049-9644, DOI: 10.1016/ J.BIOCONTROL.2016.05.010

**(Cont. next page)**

- XIANLING JI: "Biological control against bacterial wilt and colonization of mulberry by an endophytic Bacillus subtilis strain : Biological control against bacterial wilt", FEMS MICROBIOLOGY ECOLOGY, vol. 65, no. 3, 10 July 2008 (2008-07-10), NL, pages 565 - 573, XP093157433, ISSN: 0168-6496, DOI: 10.1111/j.1574-6941.2008.00543.x

- SOTOYAMA, K. ET AL.: "Suppression of bacterial with of tomato by soil amendment with mushroom compost containing Bacillus amyloliquefaciens IUMC7.", J. GEN. PLANT PATHOL., vol. 83, 2017, pages 51 - 55, XP036125767, DOI: 10.1007/s10327-016-0690-7

**Description**

Technical Field

The present invention relates to a soil-borne plant disease control agent that is sprayed to the aboveground part of a plant to effectively control bacterial wilt and other plant diseases caused by infection of the root, and/or a bacterial strain of genus Bacillus that can be cultured in a shorter time period than traditional strains.

Background Art

Control of crop diseases (plant diseases) caused by pathogenic bacteria is a very important issue among farmers around the globe. Cultural control and use of antimicrobial agents are traditional approaches to the management of plant diseases. However, some bacterial plant diseases are difficult to control by traditional methods, including cultural control and antimicrobial agents.

An example of such plant diseases is bacterial wilt, which is a soil-borne disease caused by Ralstonia solanacearum species complex, a soil-borne bacterium. When plants such as tomatoes, eggplants, peppers, and potatoes are infected with bacterial wilt pathogens, they wilt rapidly, resulting in a significant loss of yield. Ralstonia solanacearum is a widely distributed pathogen, mainly in tropical, subtropical, and temperate regions of the world, infecting over 200 plant species, particularly the Solanaceae plants, and damaging the crops. Indeed, bacterial wilt is one of the serious, important issues in farming.

To date, a large number of control techniques against bacterial wilt disease have been proposed. Examples of such techniques currently in use include use of chemical pesticides, soil disinfection such as by solar disinfection or reductive disinfection, and cultivation or breeding of resistant rootstock varieties. The bacterial wilt-causing bacteria are able to survive for prolonged time periods deep in the soil (about 50 cm to 1 m from surface) even when the soil surface is successfully disinfected by means of, for example, soil disinfection. Currently, no soil disinfectant is available that can reach into the soil this deep, and complete removal of bacteria from soil remains a difficult task indeed. The resistance of resistant varieties is not perfect either, and fails to exhibit sufficient effect depending on the environmental conditions. There has been proposed another type of pesticide, for example, such as the bacterial wilt resistance inducer (PTL 1). However, the efficacy of such pesticides is also not sufficient.

As a countermeasure, a control method is proposed that makes use of a biopesticide to inhibit the development of plant diseases through antagonistic action against pathogens present in the soil. Control of bacterial wilt-causing bacteria with microbial pesticides has been studied with regard to strains of bacteria such as Pseudomonas and Bacillus bacterial strains (NPL 1). However, control by such biopesticides is still not sufficiently effective. There have been attempts to control bacterial wilt disease by feeding compost containing different microorganisms. However, the effectiveness of this method is unclear, and many of the previous attempts have failed. Because the foregoing methods show effects by treating the soil, the antagonistic microorganisms must colonize in the soil, the rhizosphere, and the plant. However, it is often difficult to consistently produce a stable effect in widely changing conditions of soil. Another difficulty is that colonization and proliferation are difficult to achieve with strains that are not pathogenic to plant. Indeed, this is one of the reasons for the previous failure to establish a control method using biopesticides.

Given these technical backgrounds, the industry requires development and commercialization of a soil-borne disease control agent that makes use of a microorganism capable of colonizing and proliferating in the soil or plant without being pathogenic to plant but showing sufficient levels of basic activity, and that can be safely used as a pesticide with the effect to sufficiently control, both practically and stably, bacterial wilt and other soil-borne plant diseases that are difficult to control by the traditional approach.

Biol. control 2016, 100, 63-69 discloses the use of *Lactobacillus plantarum* for the treatment of cabbage against bacterial soft rot. This document is silent on the use of *Fictibacillus solisalsi* or *Bacillus timonensis* for the treatment of bacterial soft rot.

FEMSMicrobiol. Ecol.2008, 65, 565-573 discloses the use of *Bacillus subtilis* for the treatment of bacterial wilt. This document is silent on the use of *Fictibacillus solisalsi* or *Bacillus timonensis* for the treatment of bacterial wilt.

Citation List

Patent Literature

PTL 1: JP-A-2012-211124

Non Patent Literature

NPL 1: Chemistry and Biology, Kagaku to Seibutsu, Vol. 51 (2013), No. 8, p. 541 to 547

Summary of Invention

Technical Problem

It is an object of the present invention to provide a non plant-pathogenic strain that can stably exhibit a soil-borne plant disease control effect at the actual site of crop production, and that can be safely used as a biopesticide for plants, and to provide a soil-borne plant disease control agent using such a strain, among others.

Because bacterial wilt is diseases caused by bacteria inhabiting the soil, a soil treatment is often performed against bacterial wilt such as by irrigation or fumigation. However, the soil treatment has a number of disadvantages, as discussed above.

It is accordingly another object of the present invention to provide a novel and effective method of treatment, and novel and effective components that can overcome such disadvantages.

Solution to Problem

In order to achieve these objects, the present inventors studied the foregoing issues from different angles, and turned our attention to a microbial treatment among various treatment options. After further extensive studies, it was found that a previously unreported novel strain of genus Bacillus, despite being a soil disease, can sufficiently and effectively control the soil-borne diseases bacterial wilt not by soil treatment but by indirect application to aboveground structures of crop, for example, stems and leaves, with or without a soil treatment.

To put it another way, intensive studies conducted by the present inventors to achieve the foregoing objects led to the finding that a previously unreported novel strain of genus Bacillus can produce practical effects, such as control of soil-borne plant diseases, by being sprayed to stems and leaves of a plant, without directly contacting the soil. The novel Bacillus strain was found to be safe to use at the actual site of crop production. It was also found that the novel strain requires a shorter culture time than plant disease control agents containing traditional Bacillus bacteria, and enables fast production of a control agent containing the novel strain as an active component. The present invention was completed on the basis of these findings.

Specifically, embodiments of the present invention are as follows.

(1) A novel Bacillus sp. G4L1 bacterial strain (NITE BP-03204).
(2) A novel Bacillus timonensis G5S1 bacterial strain (NITE BP-03206).
(3) A novel Fictibacillus solisalsi G5L2 bacterial strain (NITE BP-03205).
(4) The strain according to any one of (1) to (3), wherein the strain is capable of controlling a plant disease by being applied to at least one of a plant seed, a plant root, a stem and leaf portion of a plant, a plant support, a nutrient solution, and soil.
(5) The strain according to any one of (1) to (3), wherein the strain has a high growth rate.
(6) A plant disease control agent comprising one or more of the strains of (1) to (3) or a culture thereof as an active component.
(7) The agent according to (6), wherein the agent is capable of controlling plant diseases by being applied to at least one of a plant seed, a plant root, a stem and leaf portion of a plant, a plant support, a nutrient solution, and soil.
(8) The agent according to (6) or (7), wherein the agent is capable of controlling diseases of at least one of vegetables, fruit trees, and gramineous plants.
(9) The agent according to any one of (6) to (8), wherein the agent is capable of controlling diseases of Solanaceae and/or Brassica plants.
(10) The agent according to any one of (7) to (9), wherein the agent is capable of controlling a soil-borne plant disease by being applied to stems and leaves of a plant.
(11) The agent according to (10), wherein the agent is capable of controlling bacterial wilt of vegetables and/or bacterial soft rots of vegetables.
(12) A plant disease control method comprising contacting or mixing viable bacteria of one or more of the strains of (1) to (3) or a culture containing said viable bacteria with a plant seed, a plant root, an aboveground part of a plant, a plant cultivation support, a nutrient solution, or soil.
(13) The method according to (12), wherein the method controls diseases of at least one of vegetables, fruit trees, and gramineous plants.
(14) The method according to (12) or (13), wherein the method controls diseases of Solanaceae and/or Brassica plants.

(15) The method according to any one of (12) to (14), wherein the method controls soil-borne plant diseases by being applied to stems and leaves of a plant.

(16) The method according to any one of (12) to (15), wherein the method controls bacterial wilt of vegetables and/or bacterial soft rot of vegetables.

Other embodiments of the present invention are as follows.

(17) A novel Bacillus sp. G4L1 bacterial strain (NITE BP-03204).

(18) A novel Bacillus timonensis G5S1 bacterial strain (NITE BP-03206).

(19) A novel Fictibacillus solisalsi G5L2 bacterial strain (NITE BP-03205).

(20) The strain according to any one of (17) to (19), wherein the strain is capable of controlling soil-borne plant diseases by being applied to stems and leaves of a plant and has a high growth rate.

(21) A plant disease control agent comprising one or more of the strains of (17) to (20) or a culture thereof as an active component.

(22) The agent according to (21), wherein the agent is a soil-borne plant disease control agent.

(23) The agent according to (22), wherein the agent is a vegetable wilt control agent.

(24) The agent according to any one of (21) to (23), wherein the agent is a disease control agent for Solanaceae plants.

(25) A plant disease control method comprising contacting viable bacteria of one or more of the strains of (17) to (20) or a culture containing said viable bacteria with a plant and/or soil (particularly, the rhizosphere).

(26) The method according to (25), wherein the method controls soil-borne plant diseases.

(27) The method according to (26), wherein the method controls bacterial wilt of vegetables.

(28) The method according to any one of (25) to (27), wherein the method controls a disease of Solanaceae plants.

(29) A method for controlling bacterial wilt of vegetables, a soil-borne plant disease, wherein the method produces a control effect only by spraying the vegetable wilt control agent of (23) to an aboveground part of a plant.

(30) The strain according to any one of (17) to (20), wherein the strain has a high growth rate, and grows in a short time period of about 10 hours in terms of a culture time needed to provide a measured absorbance value at 600 nm of 3.00 to 3.50, as opposed to at least about 15 hours required by traditional Bacillus bacteria.

(31) The strain according to (30), wherein the strain requires 9 to 12 hours, specifically 9 to 11 hours, more specifically 9 to 10 hours of culture time before a measured absorbance value at 600 nm reaches 3.00 to 3.50, as opposed to 14 to 16 hours required by traditional Bacillus bacteria.

(32) A method for fast production of a control agent of (21) or (22), comprising culturing the fast growing strain of any one of (17) to (20), (30), and (31) to obtain the strain and/or a culture thereof, and producing a controlling agent containing the strain or the culture, both in a short time period.

Advantageous Effects of Invention

According to the present invention, a soil-borne plant disease control agent can be provided that can be safely used at the actual site of crop production by being sprayed to the aboveground part of a plant to produce marked effects, both practically and stably, for the control of bacterial wilt and other soil-borne plant diseases that are difficult to control with the traditional approach. Another advantage is that a soil-borne plant disease control agent of the present invention requires a shorter culture time than soil-borne plant disease control agents containing traditional Bacillus bacteria.

Brief Description of Drawings

[Fig. 1] FIG. 1 shows a picture of a G4L1 strain-treated group after 14 days from inoculation of a causative bacterium of tomato bacterial wilt in a test conducted to confirm the effectiveness to control bacterial wilt of tomato in Example 1. The test was conducted with five pots to compare individual differences.

[Fig. 2] FIG. 2 shows a picture of an untreated group after 14 days from inoculation of a causative bacterium of tomato bacterial wilt in a test conducted to confirm the effectiveness to control bacterial wilt of tomato in Example 1.

[Fig. 3] FIG. 3 represents the method used for inoculation in Example 2.

[Fig. 4] FIG. 4 shows growth curves of four strains of Bacillus bacteria, including the G4L1 strain.

Description of Embodiments

Diverse studies conducted by the present inventors to achieve the foregoing objects drew our attention to a microbial treatment, and a search was conducted in a range of microorganisms. The studies found that the bacterial strains successfully separated by the present inventors are novel strains that were not known in the past, and that the novel strains have a new and useful effect against microorganisms that cause bacterial wilt disease. After investigations of the method of application, it was also found, rather unexpectedly, that the novel strains can control soil-borne wilt-causing bacteria not by soil treatment but by indirect application to aboveground structures of plant, for example, such as stems and leaves.

That is, the present invention has found that bacterial wilt can be controlled using newly separated strains of bacteria, not by soil treatment but by spraying to stems and leaves. These findings were systematically combined, and the present invention was completed after further studies based on this information.

In the present invention, a strain belonging to genus Bacillus or Fictibacillus is used as an active component of, for example, a control agent for soil-borne plant disease. As used herein, "soil-borne plant disease" refers to damage caused by a pathogen infecting the underground part of a useful plant. Here, damage is caused mostly by pathogens inhabiting the soil; however, "soil-borne plant disease" is not limited to damage caused by such pathogens.

Though the descriptions in this specification are based primarily on control of bacterial wilt disease, the present invention is equally effective for other soil-borne plant diseases such as bacterial soft rots.

Preferably, the strains used in the present invention are G4L1 strain of genus Bacillus, G5S1 strain of Bacillus timonensis, or G5L2 strain of Fictibacillus solisalsi, or a mutant strain of any of these strains, or a strain retaining properties similar to the properties of these strains. Here, the G4L1 strain of genus Bacillus, G5S1 strain of Bacillus timonensis, and G5L2 strain of Fictibacillus solisalsi are strains isolated from tomato leaves in the campus of Gifu University (1-1, Yanagido, Gifu, Japan), and that have been identified as non plant-pathogenic novel strains of Bacillus and Fictibacillus bacteria after studies of bacteriologic properties and phylogenetic studies of the genome sequence of the 16S rRNA gene. The G4L1, G5S1, and G5L2 strains have been shown to have the following bacteriologic properties by tests conducted using an API20NE Kit (available from Sysmex bioMerieux Co., Ltd.).

<G4L1 Strain>

G4L1 strain has the following bacteriologic properties.

(A) Morphological properties

Morphology: Rod-shaped
Size: 0.9 to 1.0 $\mu$m $\times$ 2.0 to 8.0 $\mu$m
Mobility: Absent

(B) Culture properties

Color of colony: Pale yellow to pale greenish yellow
Colony morphology: Irregular; elevation: flat; margin: undulate

(C) physiological properties

Gram staining: Positive
Optimum growth pH: Neutral range (pH 6.5 to 8.0)
Optimum growth temperature: 35°C
Nitrate reduction: +
Indole production (triptophan): -
Glucose fermentation: -
Arginine dihydrolase: +
Urease: -
Hydrolysis ($\beta$-glucosidase): +
Hydrolysis (protease): +
Assimilation (glucose): +
Assimilation (arabinose): +
Assimilation (mannose): +
Assimilation (mannitol): +
Assimilation (N-acetyl-glucosamine): +
Assimilation (maltose): +
Assimilation (potassium gluconate): +
Assimilation (capric acid): -

Assimilation (adipic acid): +
Assimilation (maleate): +
Assimilation (trisodium citrate): +
Assimilation (phenyl acetate): +

G4L1 strain is unique in that this strain, in addition to its desirable effect to control bacterial wilt disease, enables control of soil-borne plant disease, specifically, bacterial wilt and bacterial soft rots, simply by treating the aboveground part of a plant.

For species identification, G4L1 strain was analyzed by genome analysis, as follows.

A DNA barcode was created from DNA, 400 bases long, extracted from G4L1 strain. After creating a library using the Ion PGM® Hi-Q® View OT2 Kit-400 (Thermo Fisher SCIENTIFIC), a template was prepared using the Ion OneTouch® 2 System (Thermo Fisher SCIENTIFIC), a pretreatment device for next-generation sequencer. The template was sequenced by using the next-generation sequencer Ion PGM® with the Ion PGM Sequencing Hi-Q View Kit (Thermo Fisher SCIENTIFIC) and Ion 318 Chip Version 2 (Thermo Fisher SCIENTIFIC). After checking the quality of the base sequence, the adapter was removed, and the genome was sequenced by de novo assembly, using CLC Genomics Workbench Version 12 (QIAGEN) analysis software. To identify species, the sequence was analyzed by ANI analysis by comparing the whole genome with the genome of related species, using the TrueBac ID System Version 1.92 (Chunlab).

The sequence was also analyzed by DNA-DNA hybridization with related reference strains and standard strains for calculation of GGDC (formula 2) values, using the BLAST+ method (Camacho et al. 2009) with a Genome-to-Genome Distance Calculator ver 2.1 web server (Leibniz Institute DSMZ).

Table 1 shows the results of genome analysis. The ANI was 95% or less against all related reference strains and standard strains, and the GGDC (value of formula 2) was 27.3 to 56.3% relative to the related reference strains and standard strains. There are reports that a strain can be classified as a new species when the ANI value is 95% or less (reference document 1: Proc. Natl. Acad. Sci. USA, November 10, 2009 106(45) 19126-19131), and when the GGDC (value of formula 2) is 70% or less (reference document 2: Stand Genomic Sci February 28, 2010, 2(1) 117-134; reference document 3: Int J Syst Evol Microbiol January 1, 2007, 57 (1) 81-91). The G4L1 strain, satisfying these numbers, should classify itself as a new species.

[Table 1]

| Related reference strains, standard strains | ANI (%) | Value of GGDC (formula 2) (%) |
|---|---|---|
| Bacillus pseudomycoides strain AFS092012 | 88.31 | 33.6 |
| Bacillus pseudomycoides strain DSM 12442(T) | 91.06 | 42.0 |
| Bacillus cereus strain AFS028441 | 94.42 | 56.3 |
| Bacillus cereus strain Rock3-44 | 91.66 | 43.9 |
| Bacillus sp. strain AFS023182 | 90.88 | 41.1 |
| Bacillus paramycoides NH24A2(T) | 86.50 | 28.1 |
| Bacillus clarus strain BHP DJ93 | 86.91 | 29.5 |
| Bacillus nitratireducens strain 4049 (T) | 86.20 | 27.3 |
| Bacillus cereus strain BAG2X1-1 | 86.44 | 27.5 |
| Bacillus proteolyticus strain TD42 (T) | 86.34 | 27.8 |
| Bacillus bingmayongensis strain FJAT-13831 (T) | 90.73 | 40.6 |
| Bacillus cereus strain AFS096926 | 86.46 | 27.9 |
| Bacillus cereus strain HuA4-10 | 86.39 | 27.9 |
| Bacillus cereus strain BAG6X1-2 | 86.43 | 28.0 |
| Bacillus mycoides strain ATCC 6462(T) | 86.25 | 27.7 |
| Bacillus cereus strain BAG5X1-1 | 86.42 | 27.7 |

<G5S1 Strain>

G5S1 strain has the following bacteriologic properties.

(A) Morphological properties

Morphology: Rod-shaped
Size: 0.8 to 1.3 μm × 2.5 to 5.5 μm
Mobility: Present

(B) Culture properties

Color of colony: Pale reddish yellow to pale yellow (semitransparent)
Colony morphology: Circular; elevation: convex; margin: entire

(C) Physiological properties

Gram staining: Positive
Optimum growth pH: Neutral range (pH 6.5 to 7.5)
Optimum growth temperature: 40°C
Nitrate reduction: +
Indole production (triptophan): -
Glucose fermentation: -
Arginine dihydrolase: -
Urease: -
Hydrolysis (β-glucosidase): +
Hydrolysis (protease): -
Assimilation (glucose): +
Assimilation (arabinose): -
Assimilation (mannose): -
Assimilation (mannitol): -
Assimilation (N-acetyl-glucosamine): +
Assimilation (maltose): +
Assimilation (potassium gluconate): -
Assimilation (capric acid): -
Assimilation (adipic acid): -
Assimilation (maleate): -
Assimilation (trisodium citrate): -
Assimilation (phenyl acetate): -

G5S1 strain is unique in that this strain, in addition to its desirable effect to control bacterial wilt disease, enables control of soil-borne plant disease, specifically, bacterial wilt and bacterial soft rots, simply by treating the aboveground part of a plant.

<G5L2 Strain>

G5L2 strain has the following bacteriologic properties.

(A) Morphological properties

Morphology: Rod-shaped
Size: 0.8 to 1.3 μm × 3.4 to 9.0 μm
Mobility: Present

(B) Culture properties

Color of colony: Pale greenish yellow
Colony morphology: circular; elevation: umbonate; margin: entire

(C) Physiological properties

Gram staining: positive

Optimum growth pH: Neutral range (about pH 6.5)
Optimum growth temperature: 40°C
Nitrate reduction: -
Indole production (triptophan): -
Glucose fermentation: -
Arginine dihydrolase: -
Urease: -
Hydrolysis (β-glucosidase): +
Hydrolysis (protease): +
Assimilation (glucose): +
Assimilation (arabinose): +
Assimilation (mannose): +
Assimilation (mannitol): +
Assimilation (N-acetyl-glucosamine): +
Assimilation (maltose): +
Assimilation (potassium gluconate): +
Assimilation (capric acid): -
Assimilation (adipic acid): +
Assimilation (maleate): +
Assimilation (trisodium citrate): -
Assimilation (phenyl acetate): +

G5L2 strain is unique in that this strain, in addition to its desirable effect to control bacterial wilt disease, enables control of soil-borne plant disease, specifically, bacterial wilt and bacterial soft rots, simply by treating the aboveground part of a plant.

G4L1, G5S1, and G5L2 strains have been internationally deposited at The National Institute of Technology and Evaluation, NITE Patent Organism Depositary (2-5-8, Kazusa-Kamatari, Kisarazu-shi, Chiba, 292-0818, Japan; April 20, 2020) with the deposition numbers NITE BP-03204, NITE BP-03206, and NITE BP-03205, respectively.

Any medium can be used for culture of the strains belonging to genus Bacillus or Fictibacillus, provided that it allows the strains to grow. Examples include common media such as bouillon medium, and media containing glucose, peptone, and yeast extract. The medium may be a liquid medium, or a solid medium such as an agar-supplemented slant or plate medium.

As a carbon source of the medium, any source can be utilized that can be assimilated by strains belonging to genus Bacillus or Fictibacillus. Specific examples include various synthetic and natural carbon sources that can be utilized by strains of genus Bacillus, such as glucose, arabinose, mannose, starch hydrolysate, and molasses. The nitrogen source in medium may be chosen from a variety of synthetic and natural products that can be utilized by the strains, including, for example, organic nitrogen-containing products such as peptone, meat extract, yeast extract, and soybean meal. Additionally, trace amounts of nutrient sources may be added according to the customary method of microbial culture, as required. Examples of such nutrient sources include inorganic salts such as common salt and phosphates; metal salts such as calcium, magnesium, and iron salts; vitamins, amino acids, and nucleic acid-related substances. It is also possible to add an additive, for example, such as a defoaming agent, as required.

The strains belonging to the genus Bacillus or Fictibacillus may be cultured under aerobic conditions, for example, such as shaking culture or aeration culture. The culture conditions are not limited to these, and the strains are cultured for 0.5 to 2 days, preferably 1 to 1.5 days at a temperature of 20 to 45°C, preferably 30 to 40°C, at pH 5 to 8, preferably 6 to 7.

Another important characteristic of G4L1, G5S1, and G5L2 strains is the fast growth rate. The culture time specified above, specifically 0.5 days (12 hours) or longer, merely provides a rough indication of culture time with which the cell concentration reaches a sufficient level, and a practical cell concentration can be obtained with an even shorter culture time, for example, about 7 to 10 hours, though it depends on factors such as the scale of culture. Accordingly, the culture conditions do not exclude such short culture times.

Requiring less time for culture of active component microorganisms means a shorter production time for a microorganism control agent of the present invention. This makes the present invention beneficial for fast production of a microorganism control agent. The risk of contamination during culture also can be greatly reduced.

After being cultured in the manner described above, the strains belonging to the genus Bacillus and/or Fictibacillus can be used as an active component of, for example, a soil-borne plant disease control agent, in the form of a culture containing viable bacteria of these strains, without separating the strains from the culture. Alternatively, the viable bacteria may be separated from the culture using an ordinary method, for example, such as by membrane separation or centrifugation, and the separated bacteria, after optional washing, may be used as an active component as it is or after treatment (for example, as a mixture with other components). It is also possible to use the bacterial culture or separated viable bacteria in the form of a dried product obtained by using a technique such as freeze drying or spray drying, or in the form of a diluted product obtained by diluting a liquid or solid of the cultured bacteria or separated bacteria. The cultured bacteria or separated bacteria also may be used in the form of various preparations produced by mixing various additives using the traditional methods of producing pesticide formulations. Examples of such preparations include granular formulations, emulsions, wettable powders, and flowable formulations.

The concentration of viable bacteria contained in a soil-borne plant disease control agent of the present invention is not particularly limited, as long as the soil-borne plant disease control agent can produce the desired effect. However, because a bacterial concentration that is too low often fails to produce sufficient results, and a bacterial concentration that is too high is usually wasteful of bacteria, the bacterial concentration is appropriately adjusted in a range of $1 \times 10^5$ to $1 \times 10^{11}$ cfu/ml, preferably $1 \times 10^6$ to $1 \times 10^{10}$ cfu/ml, in the case of, for example, a liquid preparation. As used herein, "cfu" means colony forming unit. When using a culture, the culture may be appropriately designed within these ranges of viable bacteria concentration. Instead of cfu, the bacterial concentration may be represented by absorbance at 600 nm ($OD_{600}$). For example, an $OD_{600}$ value of 1.0 under 30°C, 200 rpm, 24-hour shake culture conditions using NB medium roughly corresponds to $2.3 \times 10^7$ cfu/ml in the case of Bacillus sp. G4L1 strain, $1.4 \times 10^7$ cfu/ml in the case of Bacillus timonensis G5S1 strain, and $1.6 \times 10^8$ cfu/ml in the case of Fictibacillus solisalsi G5L2 strain. However, these values depend on the state of strains and culture conditions, and the measured value does not necessarily correspond to these values.

The present invention enables control of plant disease, for example, bacterial wilt of Solanaceae plants (Solanaceae vegetables such as eggplant, tomato, green pepper, paprika, and potato) and Cucurbitaceae plants (Cucurbitaceae vegetables such as cucumber and bitter gourd) caused by plant pathogenic bacteria of Ralstonia solanacearum species complex. As used herein, "control" means circumventing these and other plant diseases by preventing, for example, infection of useful plants by bacteria that cause soil-borne plant diseases.

A plant disease control agent according to the present invention may be applied as it is, or after being diluted with, for example, water. Use of the plant disease control agent as a pesticidal formulation is not particularly limited, and, for example, the plant disease control agent may be directly applied to crops and seeds by being sprayed to crops and seeds or by soaking crops or seeds in the plant disease control agent. As another example, the plant disease control agent may be sprayed onto soil, or may be added to water or fertilizers added to crops and soil. As yet another example, the plant disease control agent may be applied to farm equipment. Preferably, the plant disease control agent is directly sprayed to crops. That is, a bacterial plant disease control agent according to the present invention inhibits various soil-borne plant diseases by being present on a plant, for example, on the root, stems, leaves, or seeds of a plant, or in the soil used for cultivation.

A unique feature of the present invention is that the present invention enables control of soil bacteria by being sprayed not on soil but on stems and leaves, that is, by treating the aboveground part of crops. A soil treatment is a laborious procedure involving irrigation, and requires a large quantity of pesticidal formulation, posing an environmental risk of causing contamination of water and surrounding environments. The present invention, enabling control by means of spraying to stems and leaves, can greatly reduce such a risk, and is highly desirable.

Because bacterial wilt is caused by soil bacteria, the natural reasoning would be to control the disease by soil treatment. Contrary to such common sense in the art, the present invention enables control by a less laborious method of treating only the aboveground part of a plant while still producing a sufficient bacterial wilt control effect. This makes the present invention highly valuable in technology, and the present invention can be fully expected to produce a high economic return.

A pesticidal formulation of the present invention is used in amounts that depend on factors such as the type of the crop of interest, the type of the disease to be controlled, the method of application, patterns of disease occurrence, extent of damage, environmental conditions, and the form of formulation. Accordingly, no specific amounts are set, and the pesticidal formulation is preferably used in appropriately adjusted amounts. As an example, in the case of a liquid formulation, the pesticidal formulation is used in an amount of 30 ml to 1 L, preferably 50 ml to 1 L per stock of a crop plant. The time to use the pesticidal formulation also depends on factors such as the type of the disease to be controlled, and the form of pesticidal formulation. Preferably, the pesticidal formulation is applied at an appropriate time within around 2 weeks of planting. In the present invention, there is no concern about the emergence of resistant bacteria, and the microbial pesticide of the present invention can be continuously used for several days, or can be used also for monocropping.

The present invention, including a soil-borne plant disease control agent according to the present invention, may be used with other fertilizers or agrichemicals, for example, such as germicides, antiviral agents, insecticides, miticides, nematicides, synergists, attractants, herbicides, and plant growth regulators, as required. In this case, the soil-borne plant disease control agent may be applied after being mixed with these additional components under conditions that have only little effect on the active component strain, or these may be applied at the same or different times.

The following compounds are non-limiting examples of known germicides (germicidal active components) and disease control agents that can be used as a mixture or in combination with a soil-borne plant disease control agent of the present invention.

Germicidal Active Components and Disease Control Agents:

Agrobacterim radiobacter, azaconazole, acibenzolar-S-methyl, azoxystrobin, anilazine, amisulbrom, aminopyrifen, ametoctradin, aldimorph, isotianil, isopyrazam, isofetamid, isoflucypram, isoprothiolane, ipconazole, ipflufenoquin, ipfentrifluconazole, iprodione, iprovalicarb, iprobenfos, imazalil, iminoctadine-albesilate, iminoctadine-triacetate, imibenconazole, inpyrfluxam, imprimatin A, imprimatin B, edifenphos, etaconazole, ethaboxam, ethirimol, ethoxyquin, etridiazole, enestroburin, enoxastrobin, epoxiconazole, organic oils, oxadixyl, oxazinylazole, oxathiapiprolin, oxycarboxin, oxine-copper, oxytetracycline, oxpoconazole-fumarate, oxolinic acid, copper dioctanoate, octhilinone, ofurace, orysastrobin, o-phenylphenol, kasugamycin, captafol, carpropamid, carbendazim, carboxin, carvone, Candida oleophila, Candida saitoana, quinoxyfen, quinofumelin, chinomethionat, captan, quinconazole, quintozene, guazatine, cufraneb, coumethoxystrobin, coumoxystrobin, Gliocradium catenulatum, Cryptococcus albidus, kresoxim-methyl, clozylacon, Clonostachys rosea, chlozolinate, chloroinconazide, chlorothalonil, chloroneb, Chaetomium cupreum, Coniothyrium minitans, cyazofamid, diethofencarb, diclocymet, dichlofluanid, dichlobentiazox, diclomezine, dicloran, dichlorophen, dithianon, diniconazole, diniconazole-M, zineb, dinocap, dipymetitrone, diphenylamine, difenoconazole, cyflufenamid, diflumetorim, cyproconazole, cyprodinil, simeconazole, dimethirimol, dimethyl disulfide, dimethomorph, cymoxanil, dimoxystrobin, Pseudozyma flocculosa, Pseudomonas aureofaciens, Pseudomonas chlororaphis, Pseudomonas syringae, Pseudomonas flurorescens, Pseudomonas rhodesiae, ziram, silthiofam, Zucchini yellow mosaic virus-WK, streptomycin, Streptomyces griseoviridis, Streptomyces lygicus, spiroxamine, sedaxane, seboctylamine, zoxamide, solatenol, dazomet, Talaromyces flavus, tiadinil, thiabendazole, thiram, thiophanate, thiophanate-methyl, thifluzamide, thiram, tecnazene, tecloftalam, tetraconazole, debacarb, tebuconazole, tebufloquin, terbinafine, dodine, dodemorph, triadimenol, triadimefon, triazoxide, trichlamide, triclopyricarb, Trichoderma asperellum, Trichoderma atroviride, Trichoderma gamsii, Trichoderma stromaticum, Trichoderma harzianum, Trichoderma viride, Trichoderma virens, Trichoderma polysporum, Trichoderma lignorum, tricyclazole, triticonazole, tridemorph, triflumizole, trifloxystrobin, triforine, tolylfluanid, tolclofos-methyl, tolnifanide, tolprocarb, nabam, natamycin, naftifine, nitrapyrin, nitrothal-isopropyl, nuarimol, copper nonyl phenol sulphonate, Paenibacillus polymyxa, Barkholderia cepacia, Bacillus amyloliquefaciens, Bacillus simplex, Bacillus subtilis, Bacillus pumilus, Bacillus licheniformis, harpin protein, Variovorax paradoxus, validamycin, valifenalate, Pantoea agglomerans, picarbutrazox, bixafen, picoxystrobin, Pythium oligandrum, pydiflumetofen, bitertanol, binapacryl, hinokitiol, non-pathogenic Erwinia carotovora, non-pathogenic Rhizobium vitis, biphenyl, piperalin, hymexazol, pyraoxystrobin, pyraclostrobin, pyraziflumid, pyrazophos, pyrapropoyne, pyrametostrobin, pyriofenone, pyrisoxazole, pyridachlometyl, pyrifenox, pyributicarb, pyribencarb, pyrimethanil, pyroquilon, vinclozolin, ferbam, famoxadone, phenazine oxide, fenamidone, fenaminstrobin, fenarimol, fenoxanil, ferimzone, fenpiclonil, fenpicoxamid, fenpyrazamine, fenbuconazole, fenfuram, fenpropidin, fenpropimorph, fenhexamid, folpet, phthalide, Fusarium oxysporum, bupirimate, fuberidazole, blasticidin-S, furametpyr, furalaxyl, furancarboxylic acid, fluazinam, fluindapyr, fluoxastrobin, fluoxapiprolin, fluoxytioconazole, fluopicolide, fluopimomide, fluopyram, fluoroimide, fluxapyroxad, fluquinconazole, furconazole, furconazole-cis, fludioxonil, flusilazole, flusulfamide, flutianil, flutolanil, flutriafol, flufenoxadiazam, flufenoxystrobin, flubeneteram, flumetylsulforim, flumetover, flumorph, Phlebiopsis gigantea, proquinazid, prochloraz, procymidone, prothiocarb, prothioconazole, bronopol, propamocarb-hydrochloride, propiconazole, propineb, probenazole, bromuconazole, flometoquin, florylpicoxamid, hexaconazole, benalaxyl, benalaxyl-M, benodanil, benomyl, pefurazoate, penconazole, pencycuron, benzovindiflupyr, benthiazole, benthiavalicarb-isopropyl, penthiopyrad, penflufen, boscalid, fosetyl (including salts such as aluminum, calcium, and sodium salts), polyoxin, polycarbamate, Bordeaux mixture, mancopper, mancozeb, mandipropamid, mandestrobin, maneb, myclobutanil, Mitsuaria chitosanitabida, mineral oils, mildiomycin, methasulfocarb, metam, metalaxyl, metalaxyl-M, metarylpicoxamid, metiram, metyltetraprole, metconazole, metominostrobin, metrafenone, mepanipyrim, mefentrifluconazole, meptyldinocap, mepronil, iodocarb, laminarin, phosphorous acid and salts, copper oxychloride, silver, copper(II) acetate, cuprous oxide, copper hydroxide, potassium bicarbonate, sodium bicarbonate, sulfur, oxyquinoline sulfate, copper sulfate, (3,4-dichloroisothiazol-5-yl)methyl 4-(tert-butyl)benzoate (IUPAC Name, CAS Registry Number: 1231214-23-5), UK-2A (Code Number), dodecylbenzenesulfonic acid bisethylenediamine copper[II] complex salt (DBEDC), triphenyltin acetate (TPTA), triphenyltin chloride (TPTC), triphenyltin hydroxide (TPTH).

The following are non-limiting examples of known insecticides (insecticidal active components), miticides (miticidal active components), nematicides (nematicidal active components), and synergist compounds (synergistical active components) that can be used as a mixture or in combination with a soil-borne plant disease control agent of the present invention.

Insecticidal Active Components, Miticidal Active Components, Nematicidal Active Components, and Synergistical Active Components:

Acrinathrin, azadirachtin, azamethiphos, acynonapyr, azinphos-ethyl, azinphos-methyl, acequinocyl, acetamiprid, acetoprole, acephate, azocyclotin, abamectin, afidopyropen, afoxolaner, amidoflumet, amitraz, alanycarb, aldicarb, aldoxycarb, allethrin [including d-cis-trans-form and d-trans-form], isazophos, isamidofos, isocarbophos, isoxathion, isocycloseram, isofenphos-methyl, isoprocarb, epsilon-metofluthrin, epsilon-momfluorothrin, ivermectin, imicyafos, imidacloprid, imiprothrin, indoxacarb, esfenvalerate, ethiofencarb, ethion, ethiprole, ethylene dibromide, etoxazole, etofenprox, ethoprophos, etrimfos, emamectin, emamectin benzoate, endosulfan, empenthrin, oxazosulfyl, oxamyl, oxydemeton-methyl, oxydeprofos, omethoate, nuclear polyhedrosis virus, cadusafos, kappa-tefluthrin, kappa-bifenthrin, karanjin, cartap, granulosis virus, carbaryl, carbosulfan, carbofuran, gamma-BHC, xylylcarb, quinalphos, kinoprene, chinomethionat, enterovirus, coumaphos, cryolite, clothianidin, clofentezine, chromafenozide, chlorantraniliprole, chlorethoxyfos, chlordane, chloropicrin, chlorpyrifos, chlorpyrifos-methyl, chlorfenapyr, chlorfenvinphos, chlorfluazuron, chlormephos, chlorprallethrin, entomopoxvirus, iridovirus, cyazypyr, cyanophos, diafenthiuron, diamidafos, cyantraniliprole, cyetpyrafen, dienochlor, cyenopyrafen, dioxabenzofos, diofenolan, sigmavirus, cyclaniliprole, cycloxaprid, dicrotophos, dichlofenthion, cyclobutrifluram, cycloprothrin, dichlorvos, dicloromezotiaz, dicofol, dicyclanil, disulfoton, dinotefuran, dinobuton, cyhalodiamide, cyhalothrin [including gamma-form and lambda-form], cyphenothrin [including (1R)-trans-form], cyfluthrin [including beta-form], diflubenzuron, cyflumetofen, diflovidazin, cyproflanilide, cyhexatin, cypermethrin [including alpha-form, beta-form, theta-form, and zeta-form], dimpropyridaz, dimethyl-2,2,2-trichloro-1-hydroxyethyl phosphonate (DEP), dimethylvinphos, dimethoate, dimefluthrin, jasmone, cis-jasmone, jasmonic acid, methyl jasmonate, silafluofen, cyromazine, Steinernema carpocapsae, Steinernema kushidai, Steinernema glaseri, spidoxamat, spinetoram, spinosad, spirodiclofen, spirotetramat, spiropidion, spiromesifen, sulcofuron-sodium, sulfluramid, sulfoxaflor, sulfotep, diazinon, thiacloprid, thiamethoxam, tioxazafen, thiodicarb, thiocyclam, thiosultap, thionazin, thiofanox, thiometon, tyclopyrazoflor, tetrachlorantraniliprole, tetrachlorvinphos, tetradifon, tetraniliprole, tetramethylfluthrin, tetramethrin, tebupirimfos, tebufenozide, tebufenpyrad, tefluthrin, teflubenzuron, demeton-S-methyl, temephos, deltamethrin, terbufos, tralomethrin, transfluthrin, triazamate, triazophos, trichlorfon, Trichoderma asperellum, Trichoderma harzianum, triflumuron, triflumezopyrim, trimethacarb, tolfenpyrad, naled, nicotine, nicofluprole, nitenpyram, nemadectin, densovirus, novaluron, noviflumuron, Paecilomyces lilacinus, Barkholderia cepacia, Barkholderia rinojensis, Verticillium lecanii, hydroprene, Pasteuria nishizawae, Pasteuria penetrans, Bacillus thuringiensis, entomotoxins produced by Bacillus thuringiensis, Bacillus thuringiensis subsp. Aizawai, Bacillus thuringiensis subsp. Israelensis, Bacillus thuringiensis subsp. Kurstaki, Bacillus thuringiensis subsp. Tenebrionis, Bacillus popilliae, Bacillus licheniformis, vamidothion, parathion, parathion-methyl, halfenprox, halofenozide, bioallethrin, bioallethrin S-cyclopentenyl, bioresmethrin, bis-(2-chloro-1-methylethyl)ether (DCIP), bistrifluron, hydramethylnon, bifenazate, bifenthrin, pyflubumide, piperonyl butoxide, pymetrozine, pyraclofos, pyrafluprole, pyridaphenthion, pyridaben, pyridalyl, pyrifluquinazon, pyriprole, pyriproxyfen, pirimicarb, pyrimidifen, pyriminostrobin, pirimiphos-methyl, pyrethrine, famphur, fipronil, fenazaquin, fenamiphos, fenitrothion, fenoxycarb, fenothiocarb, phenothrin [including (1R)-trans-form], fenobucarb, fenthion, phenthoate, fenvalerate, fenpyroximate, fenbutatin oxide, fenpropathrin, fonofos, sulfuryl fluoride, butocarboxim, butoxycarboxim, buprofezin, furathiocarb, prallethrin, fluacrypyrim, fluazaindolizine, fluazuron, fluensulfone, fluopyram, sodium fluoroacetate, fluxametamide, flucycloxuron, flucythrinate, flusulfamide, fluthrin, fluvalinate [including tau-form], flupyradifurone, flupyrazofos, flupyrimin, flufiprole, flufenerim, flufenoxystrobin, flufenoxuron, fluhexafon, flubendiamide, flupentiofenox, flumethrin, fluralaner, flurimfen, prothiofos, protrifenbute, flonicamid, propaphos, propargite, prohydrojasmon, profenofos, broflanilide, profluthrin, propetamphos, propoxur, flometoquin, bromopropylate, hexythiazox, hexaflumuron, Paecilomyces tenuipes, Paecilomyces fumosoroceus, Paecilomyces lilacinus, heptafluthrin, heptenophos, permethrin, benclothiaz, benzpyrimoxan, bensultap, benzoxime, bendiocarb, benfuracarb, Pochonia chlamydosporia, Beauveria tenella, Beauveria bassiana, Beauveria brongniartii, phoxim, phosalone, fosthiazate, fosthietan, phosphamidon, phosmet, polynactins, formetanate, phorate, machine oil, malathion, milbemectin, mecarbam, mesulfenfos, methomyl, metaldehyde, metaflumizone, methamidophos, metham, methiocarb, methidathion, methyl isothiocyanate, methyl bromide, methoxychlor, methoxyfenozide, methothrin, metofluthrin, methoprene, metolcarb, mevinphos, meperfluthrin, Monacrosporium phymatophagum, Monacrosporium phymatophagum, monocrotophos, momfluorothrin, Trichoderma harzianum, litlure-A, litlure-B, aluminium phosphide, zinc phosphide, phosphine, lufenuron, rescalure, resmethrin, lepimectin, rotenone, cytoplasmic polyhedrosis virus, fenbutatin oxide, calcium cyanide, organotins, nicotine-sulfate, (Z)-11-tetradecenyl acetate, (Z)-11-hexadecenal, (Z)-11-hexadecenyl acetate, (Z)-9,12-tetradecadienyl acetate, (Z)-9-tetradecen-1-ol, (Z,E)-9,11-tetradecadienyl acetate, (Z,E)-9,12-tetradecadienyl acetate, 1,1,1-trichloro-2,2-bis (4-chlorophenyl)ethane (DDT), 1,3-dichloropropene, 2,4-dichloro-5-{2-[4-(trifluoromethyl)phenyl]ethoxy}phenyl 2,2,2-trifluoroethyl sulfoxide (IUPAC Name, CAS Registry Number: 1472052-11-1), 2,4-dimethyl-5-[6-(trifluoromethylthio)hexyloxy]phenyl-2,2,2-trifluoroethyl sulfoxide (IUPAC Name, CAS Registry Number: 1472050-34-2), 2-{2-fluoro-4-methyl-5-[(2,2,2-trifluoroethyl)sulfinyl]phenoxy}-5-(trifluoromethyl)pyridine (IUPAC Name, CAS Registry Number: 1448758-62-0), 3-chloro-2-{2-fluoro-4-methyl-5-[(2,2,2-trifluoroethyl)sulfinyl]phenoxy}-5-(trifluoromethyl)pyridine (IUPAC Name, CAS Registry Number: 1448761-28-1), 4,6-dinitro-o-cresol (DNOC), 4-fluoro-2-methyl-5-(5,5-dimethylhexyloxy]phenyl 2,2,2-trifluoroethyl sulfoxide (IUPAC Name, CAS Registry Number: 1472047-71-4), Bt proteins (Cry1Ab, Cry1Ac, Cry1Fa, Cry2Ab, mCry3A, Cry3Ab, Cry3Bb, Cry34/35Ab1), methyl eugenol, 4-(p-acetoxyphenyl)-2-butanone, (Z)-10-tetradecenyl acetate, (E,Z)-4,10-tetradecadienyl acetate, (Z)-8-dodecenyl acetate, (Z)-11-tetradecenyl acetate, (Z)-13-eicosen-10-one, 14-methyl-1-octadecene, AKD-1193 (Code Number), BCS-AA10147 (Code Number), CL900167 (Code Number), O,O-diethyl-O-[4-(dimethylsulfamoyl)phenyl]-phosphorothionate (DSP), O-ethyl-O-4-(nitrophenyl)phenyl phosphonothioate (EPN), RU15525 (Code Number), XMC, Z-13-eicosen-10-one, ZXI8901 (Code Number), F4260 (Code Number).

The following compounds are non-limiting examples of known herbicides, herbicidal active components, and plant growth regulators that can be used as a mixture or in combination with a soil-borne plant disease control agent of the present invention.

Herbicide Compounds and Herbicidal Active Components:

Ioxynil (including salts such as lithium salt, sodium salt, and octanoate), aclonifen, acrolein, azafenidin, acifluorfen (including salts such as sodium salt), azimsulfuron, asulam, acetochlor, atrazine, anisiflupurin, anilofos, amicarbazone, amidosulfuron, amitrole, aminocyclopyrachlor, aminopyralid, amiprofos-methyl, ametryn, Araujia mosaic virus, alachlor, Alternaria destruens, alloxydim (including salts such as sodium salt), ancymidol, isouron, isoxachlortole, isoxaflutole, isoxaben, isodecyl alcohol ethoxylate, isoproturon, ipfencarbazone, imazaquin, imazapic (including salts such as amine salt), imazapyr (including salts such as isopropylamine salt), imazamethabenz, imazamethabenz-methyl, imazamox, imazethapyr, imazosulfuron, indaziflam, indanofan, eglinazine-ethyl, esprocarb, ethametsulfuron-methyl, ethalfluralin, ethidimuron, ethoxysulfuron, ethoxyfen, ethoxyfen-ethyl, ethofumesate, etobenzanid, epyrifenacil, endothal-disodium, oxadiazon, oxadiargyl, oxaziclomefone, oxasulfuron, oxyfluorfen, oryzalin, Obuda pepper virus, orthosulfamuron, orbencarb, oleic acid, cafenstrole, caprylic acid, capric acid, carfentrazone-ethyl, karbutilate, carbetamide, quizalofop, quizalofop-ethyl, quizalofop-P-ethyl, quizalofop-P-tefuryl, Xanthomonas campestris, quinoclamine, quinclorac, quinmerac, citric acid, cumyluron, clacyfos, glyphosate (including salts such as sodium salt, potassium salt, amine salt, propylamine salt, isopropylamine salt, ammonium salt, isopropylammonium salt, guanidine salt, monoethanolamine salt, choline salt, BAPMA (N,N-bis-(aminopropyl)methylamine) salt, dimethylamine salt, and trimesium salt), glufosinate (including salts such as amine salt and sodium salt), glufosinate-P, glufosinate-P-sodium, clethodim, clodinafop, clodinafop-propargyl, clopyralid (including salts such as monoethanolamine salt), clomazone, chlomethoxyfen, clomeprop, cloransulam-methyl, chloramben, chloridazon, chlorimuron, chlorimuron-ethyl, chlorsulfuron, chlorthal-dimethyl, chlorthiamid, chlorphthalim, chlorflurenol-methyl, chlorpropham, chlorbromuron, chloroxuron, chlorotoluron, ketospiradox (including salts such as sodium salt, calcium salt, and ammonia salt), Colletotrichum orbiculare, Colletotrichum gloeosporioides, Colletotrichum truncatum, Chondrostercum purpureum, saflufenacil, sarmentine, cyanazine, cyanamide, diuron, diethatyl-ethyl, dioxopyritrione, dicamba (including salts such as amine salt, diethylamine salt, isopropylamine salt, diglycolamine salt, dimethylammonium salt, diolamine salt, isopropylammonium salt, auramine salt, potassium salt, trolamine salt, BAPMA (N,N-bis-(aminopropyl)methylamine) salt, choline salt, sodium salt, and lithium salt; and esters, for example, such as methyl ester), cycloate, cycloxydim, diclosulam, cyclosulfamuron, cyclopyranil, cyclopyrimorate, dichlobenil, diclofop, diclofop-P-methyl, diclofop-methyl, dichlorprop, dichlorprop-P (including salts such as dimethylammonium salt, potassium salt, sodium salt, and choline salt; and esters, for example, such as butotyl ester, 2-ethylhexyl ester, isoctyl ester, and methyl ester), diquat, diquat dibromide, dithiopyr, siduron, dinitramine, cinidon-ethyl, cinosulfuron, dinoseb (including acetate), dinoterb, cyhalofop, cyhalofop-butyl, cypyrafluone, diphenamid, difenzoquat, diflufenican, diflufenzopyr, simazine, dimesulfazet, dimethachlor, dimethametryn, dimethenamid, dimethenamid-P, simetryn, dimepiperate, dimefuron, Pseudomonas fluorescens, cinmethylin, swep, sulcotrione, sulfentrazone, sulfosate, sulfosulfuron, sulfometuron-methyl, sethoxydim, Scelerothinia minor, terbacil, daimuron, thaxtomin A, Tobacco mild green mosaic tobamovirus, Tobacco rattle virus, dalapon, thiazopyr, tiafenacil, thiencarbazone (including, for example, sodium salt, and methyl ester), tiocarbazil, thiobencarb, thidiazimin, thidiazuron, thifensulfuron, thifensulfuron-methyl, desmedipham, desmetryne, tetflupyrolimet, thenylchlor, tebutam, tebuthiuron, tepraloxydim, tefuryltrione, terbuthylazine, terbutryn, terbumeton, tembotrione, topramezone, tralkoxydim, triaziflam, triasulfuron, triafamone, tri-allate, trietazine, triclopyr, triclopyr-butotyl, triclopyr-triethylammonium, tritosulfuron, tripyrasulfone, trifludimoxazin, triflusulfuron-methyl, trifluralin, trifloxysulfuron (including salts such as sodium salt), tribenuron-methyl, tolpyralate, naptalam (including salts such as sodium salt), naproanilide, napropamide, napropamide-M, nicosulfuron, lactic acid, neburon, norflurazon, Burkholderia rinojensis, vernolate, paraquat, paraquat dichloride, halauxifen, halauxifen-benzyl, halauxifen-methyl, haloxyfop, haloxyfop-P, haloxyfop-etotyl, haloxyfop-P-methyl, halosafen, halosulfuron-methyl, bixlozone, picloram (including salts such as dichloroammonium salt, and trolamine salt), picolinafen, bicyclopyrone, bispyribac-sodium, pinoxaden, bipyrazone, bifenox, piperophos, pyraclonil, pyrasulfotole, pyrazoxyfen, pyrazosulfuron-ethyl, pyrazolynate, bilanafos, pyraflufen, pyraflufen-ethyl, pyridafol, pyrithiobac-sodium, pyridate, pyriftalid, pyributicarb, pyribenzoxim, pyrimisulfan, pyriminobac-methyl, pyroxasulfone, pyroxsulam, Phytophthora palmivora, phenisopham, fenuron, fenoxasulfone, fenoxaprop (including, for example, methyl ethyl, and isopropyl esters), fenoxaprop-P (including, for example, methyl ethyl, and isopropyl esters), fenquinotrione, fenthiaprop-ethyl, fentrazamide, fenpyrazone, phenmedipham, Phoma chenopodicola, Phoma herbarum, Phoma macrostoma, butachlor, butafenacil, butamifos, butylate, Puccinia canaliculata, Puccinia thlaspeos, butenachlor, butralin, butroxydim, flazasulfuron, flamprop (including, for example, methyl ethyl, and isopropyl esters), flamprop-M (including, for example, methyl ethyl, and isopropyl esters), primisulfuron, primisulfuron-methyl, fluazifop-butyl, fluazifop-P, fluazifop-P-butyl, fluazolate, fluometuron, fluoroglycofen-ethyl, flucarbazone-sodium, fluchloralin, flucetosulfuron, fluthiacet-methyl, flupyrsulfuron-methyl (including salts such as sodium salt, calcium salt, and ammonia salt), flufenacet, flufenpyr-ethyl, flupropanate (including sodium salt), flupoxame, flumioxazin, flumiclorac-pentyl, flumetsulam, fluridone, flurtamone, fluroxypyr (including ester forms, for example, such as butomethyl ester and meptyl ester, and salts, for example, such as sodium salt, calcium salt, and ammonia salt), flurochloridone, pretilachlor, procarbazone (including salts such as sodium salt), prodiamine, prosulfuron, prosulfocarb, propaquizafop, propachlor, propazine, propanil, propyzamide, propisochlor, propyrisulfuron, propham, profluazol, prohexadione-calcium, propoxycarbazone, propoxycarbazone-sodium, profoxydim, bromacil, brompyrazon, prometryn, prometon, bromoxynil (including ester forms, for example, such as butyric acid ester, octanoic acid ester, and heptanoic acid ester), bromofenoxim, bromobutide, florasulam, florpyrauxifen, florpyrauxifen-benzyl, hexazinone, pethoxamid, benazolin, benazolin-ethyl, penoxsulam, Pepino mosaic virus, heptamaloxyloglucan, beflubutamid, beflubutamid-M, pebulate, pelargonic acid, bencarbazone, benquitrione, benzfendizone, bensulide, bensulfuron, bensulfuron-methyl, benzobicyclon, benzofenap, bentazone, pentanochlor, pendimethalin, pentoxazone, benfluralin, benfuresate, fosamine, fomesafen, foramsulfuron, forchlorfenuron, mecoprop (including salts such as sodium salt, potassium salt, isopropylamine salt, triethanolamine salt, dimethylamine salt, diolamine salt, trolamine salt, and choline salt; and esters, for example, such as ethadyl ester, 2-ethylhexyl ester, isoctyl ester, and methyl ester), mecoprop-P-potassium, mesosulfuron (including esters, for example, such as methyl ester), mesotrione, metazachlor, metazosulfuron, methabenzthiazuron, metamitron, metamifop, metam (including salts such as sodium salt), disodium methyl arsonate (DSMA), methiozolin, methyldymuron, metoxuron, metosulam, metsulfuron-methyl, metobromuron, metobenzuron, metolachlor, metribuzin, mepiquat chloride, mefenacet, monosulfuron (including methyl ester, ethyl ester, and isopropyl ester), monolinuron, molinate, iodosulfuron, iodosulfulon-methyl-sodium, iofensulfuron, iofensulfuron-sodium, lactofen, lancotrione, linuron, rimisoxafen, rimsulfuron, lenacil, 2,2,2-trichloroacetic acid (TCA) (including salts such as sodium salt, calcium salt, and ammonia salt), 2,3,6-trichlorobenzoic acid (2,3,6-TBA), 2,4,5-trichlorophenoxyacetic acid (2,4,5-T), 2,4-dichlorophenoxyacetic acid (2,4-D) (including salts such as amine salt, diethylamine salt, triethanolamine salt, isopropylamine salt, dimethylammonium salt, diolamine salt, dodecylammonium salt, heptylammonium salt, tetradecylammonium salt, triethylammonium salt, tris(2-hydroxypropyl)ammonium salt, trolamine salt, choline salt, sodium salt, and lithium salt; and esters, for example, such as butotyl ester, 2-butoxypropyl ester, 2-ethylhexyl ester, methyl ester, ethyl ester, butyl ester, isobutyl ester, octyl ester, pentyl ester, propyl ester, isoctyl ester, isopropyl ester, meptyl ester, and tefuryl ester), 2,4-dichlorophenoxybutyric acid (2,4-DB) (including salts such as amine salt, diethylamine salt, triethanolamine salt, isopropylamine salt, dimethylammonium salt, choline salt, sodium salt, and lithium salt; and esters, for example, such as isoctyl ester), 2-amino-3-chloro-1,4-naphthoquinone (ACN), 2-methyl-4-chlorophenoxyacetic acid (MCPA) (including salts such as sodium salt, dimethylammonium salt, and choline salt; and esters, for example, such as 2-ethylhexyl ester, isoctyl ester, and ethyl ester), 2-methyl-4-chlorophenoxybutyric acid (MCPB) (including, for example, sodium salt, and ethyl ester), 4-(2,4-dichlorophenoxy)butyric acid (2,4-DB), 4,6-dinitro-O-cresol (DNOC) (including salts such as amine salt and sodium salt), (5S)-3-(3,5-difluorophenyl)-N-[rel-(3R,5R)-5-(trifluoromethylsulfonylcarbamoyl)tetrahydrofuran-3-yl]-5-vinyl-4H-isoxazole-5-carboxyamide) (IUPAC Name; CAS Registry Number: 2266183-40-6; WO2018/228986, WO2020/114934), N4-(2,6-difluorophenyl)-6-(1-fluoro-1-methyl-ethyl)-1,3,5-triazine-2,4-diamine (IUPAC Name; CAS Registry Number: 1606999-43-2; WO2014/064094, WO2015/162164), (5S)-3-(3,5-difluorophenyl)-N-[(3R)-5-(methylsulfonylcarbamoyl)-2,3-dihydrofuran-3-yl]-5-vinyl-4H-isoxazole-5-carboxyamide) (IUPAC Name; CAS Registry Number: 2266190-06-9; WO2018/228986, WO2020/114934), (5R)-3-(3,5-difluorophenyl)-5-methyl-N-[rel-(3R,5R)-5-(methylsulfonylcarbamoyl)tetrahydrofuran-3-yl]-4H-isoxazole-5-carboxyamide) (IUPAC Name; CAS Registry Number: 2266164-36-5; WO2018/228986, WO2020/114934), (5R)-3-(3,5-difluorophenyl)-N-[(3R)-5-(methoxycarbamoyl)-2,3-dihydrofuran-3-yl]-5-methyl-4H-isoxazole-5-carboxyamide) (IUPAC Name; CAS Registry Number: 2266170-31-2; WO2018/228986, WO2020/114934), 2-[2-(3,4-dimethoxyphenyl)-6-methyl-3-oxo-pyridazine-4-carbonyl]cyclohexane-1,3-dione) (IUPAC Name; CAS Registry Number: 2138855-12-4; WO2017/178582, WO2018/015476), 4-hydroxy-1-methyl-3-[4-(trifluoromethyl)-2-pyridyl]imidazolidin-2-one) (IUPAC Name; CAS Registry Number: 1708087-22-2; WO2015/059262, WO2018/015476), 6-(1-fluorocyclopentyl)-N4-(2,3,5,6-tetrafluorophenyl)-1,3,5-triazine-2,4-diamine) (IUPAC Name; CAS Registry Number: 1820807-75-7; WO2015/162164), 6-(1-fluoro-1-methyl-ethyl)-N4-(2,3,5,6-tetrafluorophenyl)-1,3,5-triazine-2,4-diamine) (IUPAC Name; CAS Registry Number: 1606999-21-6; WO2014/064094, WO2015/162164), (5S)-3-(3-fluoro-5-methyl-phenyl)-N-[rel-(3R,5R)-5-(methoxycarbamoyl)tetrahydrofuran-3-yl]-5-vinyl-4H-isoxazole-5-carboxyamide) (IUPAC Name; CAS Registry Number: 2266292-43-5; WO2018/228986, WO2020/114934), 6-(1-methylcyclobutyl)-N4-(2,3,5,6-tetrafluorophenyl)-1,3,5-triazine-4,4-diamine) (IUPAC Name; CAS Registry Number: 1607001-97-7; WO2014/064094, WO2015/162164), AE-F-150944 (Code Number), F9960 (Code Number), IR-6396 (Code Number), MCPA-thioethyl, NC-656 (Code Number), SYP-298 (Code Number), SYP-300 (Code Number), S-ethyldipropylthiocarbamate (EPTC), S-metolachlor, S-9750 (Code Number), MSMA, HW-02 (Code Number), S-523 (Code Number), and SL-1201 (Code Number).

Plant Growth Regulators:

1-Naphthylacetamide, 1-methylcyclopropene, 1,3-diphenylurea, 2,3,5-triiodobenzoic acid, 2-methyl-4-chlorophenoxybutyric acid (MCPB) [including, for example, sodium salt, and ethyl ester], 2-(naphthalene-1-yl)acetamide, 2,6-diisopropylnaphthalene, 3-[(6-chloro-4-phenylquinazoline-2-yl)amino]propane-1-ol, 4-oxo-4-(2-phenylethyl)aminobutyric acid (IUPAC Name; CAS Registry Number: 1083-55-2), 4-chlorophenoxyacetic acid (4-CPA), 5-aminolevulinic acid hydrochloride, methyl 5-(trifluoromethyl)benzo[b]thiofen-2-carboxylate, AVG (aminoethoxyvinylglycine), n-decyl alcohol (n-decanol), anisiflupurin, aviglycine, ancymidol, abscisic acid, isoprothiolane, inabenfide, indole acetic acid, indole butyric acid, uniconazole, uniconazole-P, Ecolyst, ethychlozate, ethephon, epocholeone, calcium chloride, choline chloride, oxine-sulfate, opabactin, kinetin, calcium peroxide, carvone, quinabactin, calcium formate, cloxyfonac, cloxyfonac-potassium, cloprop, chlormequat, chlormequat-chloride, chlorpropham, choline, cytokinins, oxidized glutathione, cyanamide, sodium cyanate, cyclanilide, dichlorprop (including salts such as dimethylammonium salt, potassium salt, sodium salt, and choline salt; and esters, for example, such as butotyl ester, 2-ethylhexyl ester, isoctyl ester, and methyl ester), dichlorprop-P (including salts such as sodium salt, potassium salt, and dimethylammonium salt; and 2-ethylhexyl ester), diquat, diquat dibromide, dikegulac, gibberellinic acid, gibberellin A4, gibberellin A7, dimethipin, sintofen, jasmone, cis-jasmone, jasmonic acid, methyl jasmonate, streptomycin, calcium polysulfide, daminozide, calcium carbonate, thidiazuron, decan-1-ol, triacontanol, triapenthenol, trinexapac-ethyl, tribufos, paclobutrazol, paraffin, bispyribac-sodium, hymexazol, butralin, fluthiacet-methyl, pyraflufen-ethyl, flumetralin, flurprimidol, flurenol, pronitridine, prohydrojasmon, prohexadione-calcium, heptamaloxyloglucan, 6-benzylaminopurine, pendimethalin, forchlorfenuron, formononetin, maleic hydrazide, mepiquat chloride, mefluidide, lipochitooligosaccharides (for example, lipochitooligosaccharides SP104), and calcium sulfate.

The following are non-limiting examples of known safener compounds that can be used as a mixture or in combination with a soil-borne plant disease control agent of the present invention.

Safener Compounds:

Isoxadifen, isoxadifen-ethyl, oxabetrinil, octane-1,8-diamine, cloquintocet, cloquintcet-mexyl, dietholate, cyometrinil, dichlormid, dicyclonone, cyprosulfamide, daimuron, 1,8-naphthalic anhydride, fenchlorazole, fenchlorazole-O-ethyl, fenclorim, furilazole, fluxofenim, flurazole, benoxacor, metcamifen, mephenate, mefenpyr, mefenpyr-ethyl, mefenpyr-diethyl, lower alkyl substituted benzoic acid, 2,2-dichloro-N-(1,3-dioxolan-2-ylmethyl)-N-(2-propenyl)acetamide (PPG-1292), 2-dichloromethyl-2-methyl-1,3-dioxane (MG-191), 3-dichloroacetyl-2,2,5-trimethyl-1,3-oxazolidine (R-29148), 4-dichloroacetyl-1-oxa-4-azaspiro[4.5]decane (AD-67), 4-carboxy-3,4-dihydro-2H-1-benzopyran-4-acetic acid (CL-304415, Code Number), MON4660 (Code Number), metcamifen, N1,N2-diallyl-N2-dichloroacetylglycineamide (DKA-24, Code Number), 1-bromo-4-[(chloromethyl)sulfonyl]benzene (CSB), 2-propenyl 1-oxa-4-azaspiro[4,5]decane-4-carbodithioate (MG-838, Code Number), 3-(dichloroacetyl)-2,2-dimethyl-1,3-oxazolidine (R-28725, Code Number), R-29148 (Code Number), and 1-(dichloroacetyl)azepane (TI-35, Code Number).

The following are non-limiting examples of known biopesticides that can be used as a mixture or in combination with a soil-borne plant disease control agent of the present invention.

Biopesticides:

Haplothrips brevitubus, Franklinothrips vespiformis, Diglyphus isaea, Encarsia formosa, Amblyseius cucumeris, Pseudaphycus malinus, Amblyseius womersleyi, Aphidius colemani, Eretmocerus eremicus, Aphidoletes aphidimyza, Amblyseius swirskii, Orius strigicollis, Phytoseiulus persimilis, Amblyseius degenerans, Phytoseiulus persimilis, Orius sauteri, Dacnusa sibirica, Amblyseius californicus, Chrysoperla nipponensis, and Anicetus beneficus.

The following are non-limiting examples of known agricultural materials that can be used as a mixture or in combination with a soil-borne plant disease control agent of the present invention.

Agricultural Materials:

Ethylene, a hypochlorous acid solution (only those produced by electrolysis of hydrochloric acid or an aqueous solution of potassium chloride), baking soda, vinegar, humus, humic acid, fulvic acid, seaweed extracts, polysaccharides, amino acids, microbial materials, functional components derived from animals and plants, microbial metabolites, microbial active materials, soil spreading agents, soil-systemic regulatory materials, soil-water retaining materials, and biostimulants.

The following are non-limiting examples of known agricultural fertilizer components that can be used as a mixture or in combination with a soil-borne plant disease control agent of the present invention.

Fertilizers include inorganic fertilizers and organic fertilizers. Examples include ammonium chloride, ammonium sulfate, ammonium nitrate, ammonium dihydrogenphosphate, urea ammonium nitrate, urea, nitrolime, potassium nitrate, superphosphate, double superphosphate, potassium dihydrogen phosphate, potassium chloride, potassium sulfate, potassium carbonate, potassium silicate, oil meal, fish meal, rice bran, bat guano, and fermented chicken manure.

The present invention, including a soil-borne plant disease control agent of the present invention, is also applicable to plants that have acquired properties such as pest resistance, disease resistance, and herbicide resistance by techniques such as newer breeding techniques (e.g., genetic recombination, genome editing), and artificial crossing.

As described above, viable bacteria of strains of genus Bacillus, for example, Bacillus G4L1 strain, Bacillus timonensis G5S1 strain, and/or Fictibacillus solisalsi G5L2 strain, or a culture of such viable bacteria can be used as an active component to provide a pesticidal formulation that produces a notable effect for the control of soil-borne plant disease such as bacterial wilt of vegetables while being safe to use at the actual site of crop production.

The following describes the present invention through Examples. It is to be noted that the present invention is not limited to the Examples below.

Example 1

(Test for Confirmation of Control Effect on Bacterial Wilt of Tomato)

A tomato (variety: Ponderosa) was grown to the fourth compound-leaf stage in a pot (9 cm $\times$ 9 cm) filled with a planting soil. The plant was then treated with a suspension of Bacillus sp. G4L1 strain, a suspension of Bacillus timonensis G5S1 strain, and a suspension of Fictibacillus solisalsi G5L2 strain by spraying these suspension, 50 mL each, to the stems and leaves of plant using a hand sprayer. After being kept in a 35°C greenhouse for 7 days, the tomato plant was inoculated with 50 ml of a suspension of causative bacteria of tomato bacterial wilt by supplying water from the bottom with the bacterial suspension. For comparison, some tomato stocks were treated only with a suspension of causative bacteria of tomato bacterial wilt supplied with water from the bottom using the same procedure (untreated group). The suspensions of G4L1 strain, G5S1 strain, and G5L2 strain were prepared by growing these bacterial strains by shake culture in NB medium (0.5% meat extract, 1.5% peptone, 0.5% sodium chloride, 0.5% dipotassium phosphate, pH 7.0) at 30°C, 200 rpm for 24 hours. The cells were harvested after centrifugation (4,500 $\times$ g, 15 minutes), and were suspended in sterile water and centrifuged again. This procedure was repeated twice, and the suspensions were used after adjusting the absorbance at 600 nm ($OD_{600}$) to 1.0. A suspension of causative bacteria of tomato bacterial wilt was prepared by growing the bacterial cells by shake culture in YP medium (0.5% yeast, 1.0% peptone, pH 6.8 to 7.2) for 24 hours. After centrifugation, the harvested cells were diluted with distilled water, and the suspension was used after adjusting the absorbance at 600 nm ($OD_{600}$) to 0.02 (9.2 $\times$ $10^6$ cfu/ml).

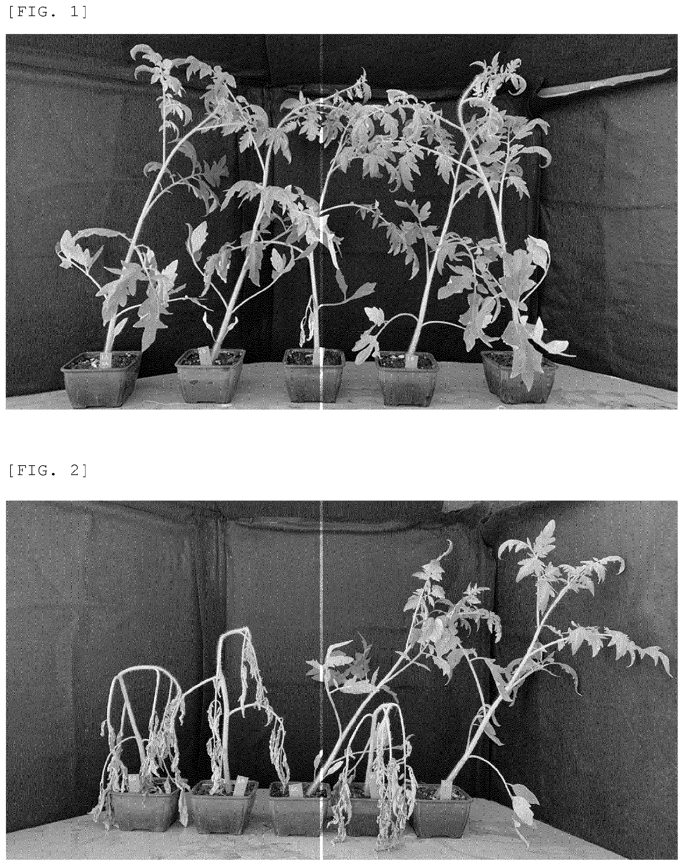

The extent of bacterial wilt of tomato was examined after 20 days from inoculation of the causative bacteria of tomato bacterial wilt, using the following index criteria. The results were used to calculate an incidence of disease and control efficacy. A picture was taken for five of the stocks from each group. The results are shown in FIGS. 1 and 2 representing photographs substituted for drawing.

<Disease Index>

0: No disease
1: Wilt occurred in some leaflets
2: Wilt occurred in less than half of compound leaves
3: Wilt occurred in at least half of compound leaves
4: Withered and died

<Incidence of Disease and Control Efficacy>

Incidence of disease = $\Sigma$(disease index $\times$ number of stocks with corresponding index)/(number of stocks examined $\times$ 4) $\times$ 100

Control efficacy = 100 - (incidence of disease of treated group/incidence of disease of untreated group) $\times$ 100

The test results are shown in Table 2 and in FIG. 1 (a photograph substituted for drawing). In contrast to the untreated group in which 2/3 of leaves developed bacterial wilt of tomato, the groups treated with G4L1 strain, G5S1 strain, and G5L2 strain had much lower percentages of diseased leaves, and the average incidence of disease was much lower than that of the untreated group. This, combined with the high control efficacy, confirmed that these strains have a strong control effect against bacterial wilt of tomato. The result for the untreated group is shown in FIG. 2 representing a photograph substituted for drawing.

[Table 2]

| Strain | Percentage of diseased leaves (%) | Incidence of disease | Control efficacy |
|---|---|---|---|
| G4L1 | 16.7 | 12.5 | 72.7 |
| G5S1 | 16.7 | 8.3 | 81.8 |
| G5L2 | 16.7 | 12.5 | 72.7 |
| Untreated | 66.7 | 45.8 | |

Example 2

(Comparative Test of Control Effect on Bacterial Wilt of Tomato Using Existing Bacillus Agents)

A tomato (variety: Ponderosa) was grown to the fourth compound-leaf stage in a pot (9 cm $\times$ 9 cm) filled with a planting soil. The plant was then treated with a suspension of Bacillus sp. G4L1 strain, a suspension of Bacillus timonensis G5S1 strain, and a suspension of Fictibacillus solisalsi G5L2 strain by spraying these suspensions, 50 mL each, to the stems and leaves of plant using a hand sprayer. The plant was also treated with existing bacillus agents Impression Clear® (manufactured by SDS Biotec K.K.), Agrocare wettable powder® (manufactured by Nippon Soda Co., Ltd.), Ecoshot® (manufactured by Kumiai Chemical Industry Co., Ltd.), and Validacin liquid formulation 5® (an antibiotic agent manufactured by Sumitomo Chemical Co., Ltd.) by spraying these comparative agents in the same fashion. After being kept in a 35°C greenhouse for 7 days, the tomato plant was inoculated with 50 ml of a suspension of causative bacteria of tomato bacterial wilt by supplying water from the bottom with the bacterial suspension. For comparison, some tomato stocks were treated only with a suspension of causative bacteria of tomato bacterial wilt supplied with water from the bottom using the same procedure (untreated group). The suspensions of G4L1 strain, G5S1 strain, and G5L2 strain were prepared by growing these bacterial strains by shake culture in NB medium (0.5% meat extract, 1.5% peptone, 0.5% sodium chloride, 0.5% dipotassium phosphate, pH 7.0) at 30°C, 200 rpm for 24 hours. The cells were harvested after centrifugation (4,500 $\times$ g, 15 minutes), and were suspended in sterile water and centrifuged again. This procedure was repeated twice, and the suspensions were used after adjusting the absorbance at 600 nm ($OD_{600}$) to 1.0. As shown in Table 3 below, the number of viable bacteria in the suspension corresponds to $2.3 \times 10^7$ cfu/ml for G4L1 strain suspension, $1.6 \times 10^8$ cfu/ml for G5S1 strain suspension, and $1.4 \times 10^7$ cfu/ml for G5L2 strain suspension. A suspension of causative bacteria of tomato bacterial wilt was prepared by growing the bacterial cells by shake culture in YP medium (0.5% yeast, 1.0% peptone, pH 6.8 to 7.2) for 24 hours. After centrifugation, the harvested cells were diluted with distilled water, and the suspension was used after adjusting the absorbance at 600 nm ($OD_{600}$) to 0.02 ($9.2 \times 10^6$ cfu/ml). For comparative agents, the number of viable bacteria was adjusted to $1.6 \times 10^8$ cfu/ml for Impression Clear, $1.6 \times 10^8$ cfu/ml for Agrocare wettable powder, and $1.6 \times 10^8$ cfu/ml for Ecoshot. The Validacin liquid formulation 5 was used in the test after being prepared into a 1:800 dilute solution.

The extent of bacterial wilt of tomato was examined after 11 days from inoculation of the causative bacteria of tomato bacterial wilt, using the disease index of Example 1, and the incidence of disease and control efficacy were calculated.

The active ingredients of the commercially available control agents are as follows.

(A) Impression Clear: Bacillus amyloliquefaciens AT-332 (product of SDS Biotec K.K.)
(B) Agrocare wettable powder: Bacillus subtilis HAI-0404 (product of Nippon Soda Co., Ltd.)
(C) Ecoshot: Bacillus subtilis D747 strain (product of Kumiai Chemical Industry Co., Ltd.)
(D) Validacin liquid formulation 5: validamycin A (product of Sumitomo Chemical Co., Ltd.)

The test results are presented in the Table 3 below. The groups treated with the suspension of Bacillus sp. G4L1 strain, the suspension of Bacillus timonensis G5S1 strain, and the suspension of Fictibacillus solisalsi G5L2 strain showed a clearly superior control effect on bacterial wilt of tomato to the comparative groups treated with the existing bacillus agents Impression Clear, Agrocare wettable powder, and Ecoshot, even with the same or fewer number of viable bacteria. The effect was also clearly superior to that produced by Validacin liquid formulation 5, an antibiotic agent known to show effect on bacterial wilt of tomato when sprayed to stems and leaves.

[Table 3]

| Tested bacterial strains, formulations | Treatment concentration | Incidence of disease | Control efficacy |
|---|---|---|---|
| G4L1 | $2.3 \times 10^7$ cfu/ml | 25 | 68.8 |
| G5S1 | $1.6 \times 10^8$ cfu/ml | 30 | 62.5 |
| G5L2 | $1.4 \times 10^7$ cfu/ml | 35 | 56.3 |
| Impression Clear | $1.6 \times 10^8$ cfu/ml | 95 | 0 |
| Agrocare wettable powder | $1.6 \times 10^8$ cfu/ml | 60 | 25.0 |
| Ecoshot | $1.6 \times 10^8$ cfu/ml | 60 | 25.0 |
| Validacin liquid formulation 5 | 1:800 dilution | 60 | 25.0 |
| Untreated | - | 80 | - |

Example 3

(Test for Confirmation of Control Effect on Bacterial Wilt of Tomato by Treatment of Aboveground Part Alone)

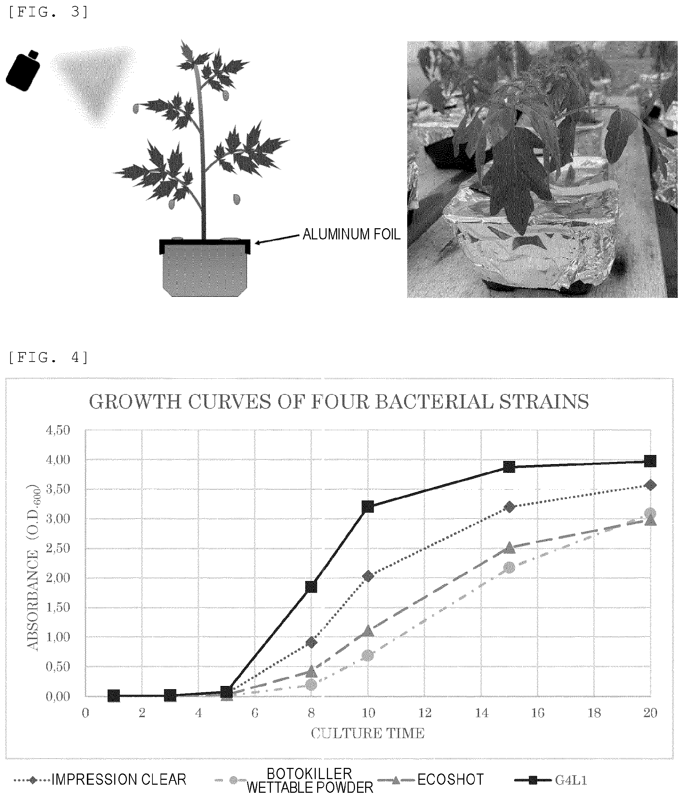

In order to confirm the effectiveness of the strains on bacterial wilt disease by indirect application to stems and leaves by means of spraying, a tomato (momotaro 8) was grown to the fourth compound-leaf stage in the same manner as in Example 1, and the ground was completely covered with an aluminum foil to prevent the strains from dropping into the soil (FIG. 3). After spraying 50 ml of a suspension of Bacillus sp. G4L1 strain to stems and leaves using a hand sprayer, the plant was completely dried in air, and the aluminum foil was removed. The plant was then kept in a 35°C greenhouse for 7 days. For inoculation, the plant was treated with a 50-ml suspension of causative bacteria of tomato bacterial wilt by supplying the suspension with water from the bottom. For comparison, a suspension of causative bacteria of tomato bacterial wilt was supplied with water from the bottom without treating the plant with the Bacillus sp. G4L1 strain (untreated group). The suspension of G4L1 strain was prepared by growing this bacterial strain by shake culture in NB medium (0.5% meat extract, 1.5% peptone, 0.5% sodium chloride, 0.5% dipotassium phosphate, pH 7.0) at 30°C, 200 rpm for 24 hours. The cells were harvested after centrifugation ($4,500 \times g$, 15 minutes), and were suspended in sterile water and centrifuged again. This procedure was repeated twice, and the suspension was used after adjusting the absorbance at 600 nm ($OD_{600}$) to 1.0. A suspension of causative bacteria of tomato bacterial wilt was prepared by growing the bacterial cells by shake culture in YP medium for 24 hours. After centrifugation, the harvested cells were diluted with distilled water, and the suspension was used after adjusting the absorbance at 600 nm ($OD_{600}$) to 0.02 ($9.2 \times 10^6$ cfu/ml).

For ten of the tomato stocks in each treated group, the extent of bacterial wilt of tomato was examined after 13 days from inoculation of the causative bacteria of tomato bacterial wilt, using the disease index of Example 1, and the incidence of disease and control efficacy were calculated.

The test results are presented in Table 4. In contrast to the untreated group in which as high as 60% of the leaves developed bacterial wilt of tomato, the incidence of disease was clearly lower in the group treated with the G4L1 strain, showing that this strain is highly effective in controlling the soil-borne bacterial wilt of tomato even when indirectly applied to stems and leaves by spraying.

[Table 4]

| Strain | Method of treatment | Incidence of disease | Control efficacy |
|---|---|---|---|
| G4L1 | Sprayed to stems and leaves in aboveground part of plant | 5.0 | 91.7 |
| Untreated | - | 60.0 | |

Example 4

(Preparation of Endospore Solution of Bacillus sp. G4L1 Bacteria)

Bacillus sp. G4L1 bacteria were inoculated to NA medium, and cultured at 30°C for 24 hours. The resulting colonies were grown in NB medium at 200 rpm, 35°C for 4 days, and were left to stand at 4°C for 2 days to promote bacteriolysis. A defoaming agent Silicone KS-66® (manufactured by Shin-Etsu Chemical Co., Ltd.) was added during culture, as appropriate.

(Test for Confirmation of Control Effect on bacterial soft rot in Napa Cabbage)

Napa cabbage was inoculated with causative bacteria of cabbage soft rots (Erwinia carotovora subsp. carotovora), and the bacteria were allowed to grow at 28°C for 48 hours in a glass-covered, moisture controlled room. Separately, a bacterial soft rot-contaminated field was created by spraying the bacteria to a cultivation field and plowing the soil 9 days before transplantation of diseased napa cabbage. For transplantation, seedlings of napa cabbage (variety: Musoh) were grown for 41 days in a 36 hole-cell tray filled with a planting soil (Yosaku®, manufactured by JCAM AGRI Co., Ltd.), and were transplanted to the soft rot-contaminated field at 30-cm intervals between plants and with an inter-row space of 40 cm. A Bacillus sp. G4L1 preparation ($5.7 \times 10^9$ cfu/g), obtained after fluidized bed granulation of the endospore solution, was diluted 250 times with water, and a spreading agent (Kumiai Kumiten®, manufactured by Kumiai Chemical Industry Co., Ltd.) was added to the dilute solution in 1/5,000th the amount. The solution so prepared was sprayed to stems and leaves with a gas atomizer after 26 days and 33 days from transplantation, 200 L/10a each time. For comparison, a dilute solution containing only the spreading agent but no preparation of Bacillus sp. G4L1 was sprayed to stems and leaves (untreated). In another group, the same spreading agent was added to a 1:500 dilute solution of an inorganic copper agent Z-Bordeaux® wettable powder commonly used for control of vegetable bacterial soft rots, and the mixture was sprayed to stems and leaves.

After 41 days from transplantation, 26 stocks of napa cabbage were collected from each group, and the extent of bacterial soft rots was examined using the following criteria. The results were used to calculate an incidence of disease and control efficacy.

<Disease Index>

  0: No disease
  1: Disease occurred in part of outer leaves
  2: Disease occurred in part of outer leaves and head leaves
  3: Disease occurred in most of head leaves, or damage occurred in greater parts of plant

<Incidence of Disease and Control Efficacy>

Incidence of disease = Σ(disease index × number of stocks with corresponding index) × 100/(number of stocks investigated × 3) Control efficacy = 100 - (incidence of disease of treated group/incidence of disease of untreated group) × 100

The results are presented in Table 5. The napa cabbage sprayed with the G4L1 preparation had a lower incidence of disease than the untreated group, which had an incidence of disease of 23.1, and the control efficacy was higher than in the group sprayed with the Z-Bordeaux® wettable powder. As demonstrated above, the Bacillus sp. G4L1 bacteria are highly effective at controlling bacterial soft rots of napa cabbage.

[Table 5]

| Tested pesticidal formulation | Incidence of disease | Control efficacy |
|---|---|---|
| G4L1preparation | 12.5 | 45.8 |
| Z-Bordeaux wettable powder | 15.7 | 31.9 |
| Untreated | 23.1 | |

The active component of the commercially available control agent above is as follows.

  (A) Z-Bordeaux wettable powder: Basic copper sulfate (Nihon Nohyaku Co., Ltd.)

Example 5

(Test for Confirmation of Proliferation Potential)

G4L1 strain was inoculated to NA medium (0.5% meat extract, 1.5% peptone, 0.5% sodium chloride, 1.5% agar, pH 7.0) at 30°C, for 24 hours. For comparison, commercially available Bacillus disease control agents Impression Clear®, Botokiller® wettable powder, and Ecoshot® were also inoculated under the same conditions. Single colonies that appeared after 24 hours were collected, and grown by shake culture in NB medium (0.5% meat extract, 1.5% peptone, 0.5% sodium chloride, 0.5% dipotassium phosphate, pH 7.0) at 30°C, 200 rpm for 24 hours. The cells were harvested after centrifugation (4,500 × g, 15 minutes), and were suspended in sterile water and centrifuged again. This procedure was repeated twice, and the suspensions were used after adjusting the absorbance at 600 nm ($OD_{600}$) to 0.1. Thereafter, 100 μl of the adjusted bacterial suspension was added to an L-shaped test tube filled with 5 ml of NB medium. To create a growth curve, the suspension was measured for absorbance at 600 nm ($OD_{600}$) while growing the bacteria by shake culture at 30°C, 70 rpm conditions using a small-scale shake culture apparatus.

The active components of the commercially available control agents above are as follows.

(A) Impression Clear: Bacillus amyloliquefaciens AT-332 (manufactured by SDS Biotech K.K.)
(B) Botokiller wettable powder: Bacillus subtilis (manufactured by Idemitsu Kosan Co., Ltd.)
(C) Ecoshot: Bacillus subtilis D747 (manufactured by Kumiai Chemical Industry Co., Ltd.)

The results of culture are presented in Table 6 and in FIG. 4 (Curves of four Cultured Strains). The growth rate of G4L1 strain was visibly faster than those of the commercially available Bacillus disease control agents, and reached the stationary phase faster than any of the commercially available control agents. G4L1 strain also had a higher absorbance than the other Bacillus disease control agents, indicating that the G4L1 strain grows faster than the conventional strains, and can be cultured in a shorter time period.

[Table 6]

| Culture time | G4L1 strain | Active component strain of Impression Clear | Active component strain of Botokiller wettable powder | Active component strain of Ecoshot |
|---|---|---|---|---|
| 1 | 0.01 | 0.01 | 0 | 0 |
| 3 | 0.01 | 0.01 | 0.01 | 0.01 |
| 5 | 0.07 | 0.06 | 0.01 | 0.03 |
| 8 | 1.84 | 0.91 | 0.19 | 0.43 |
| 10 | 3.20 | 2.03 | 0.68 | 1.11 |
| 15 | 3.87 | 3.20 | 2.17 | 2.51 |
| 20 | 3.97 | 3.57 | 3.08 | 2.98 |

As clearly demonstrated above, the strain according to the present invention has a fast growth rate, showing a sharp increase from hour 5 to hour 8, and from hour 8 to hour 10 during culture, as is clear from the absorbance data. Indeed, at hour 8 of culture, the present strain showed an absorbance that is about twice as high as, or even higher than (about) two times the absorbance of the strain that produced the best result among the conventional strains (in this example, the active component strain of Impression Clear). The absorbance was also very high at hour 10 of culture in the strain of the present invention. While the culture time needed to achieve an absorbance at 600 nm ($OD_{600}$) of 3.00 to 3.50 was 15 hours in the strain that produced the best result among the conventional Bacillus strains (in this example, the active component strain of Impression Clear), the present strain achieved this absorbance in merely 10 hours of culture time, demonstrating that the present strain has a very high growth rate.

A faster growth rate means that the active component becomes available in a shorter time period, enabling faster production for the control agent according to the present invention. In production of microbial pesticides, culture and growth of a microorganism is essentially the rate-limiting step of chemical reaction, and the control agent can be advantageously produced in a shorter time period when this step takes less time to complete. This is also beneficial in preventing contamination, aside from being advantageous in preparing formulations.

The present invention can be summarized as follows.

An object of the present invention is to provide a novel non plant-pathogenic strain that can stably exhibit a soil-borne plant disease control effect at the actual site of crop production, and that can be safely used as a biopesticide. The present invention is also intended to provide a soil-borne plant disease control agent using such a novel strain, among others.

The active component used in the present invention includes viable bacteria of novel strains of Bacillus and Fictibacillus, or a culture of these bacteria, which were not known to have control effect on bacterial plant disease and soil-borne plant disease. This has made it possible to provide a soil-borne plant disease control agent, for example, a vegetable wilt control agent. A feature of the control agent is that it can be applied (sprayed) to stems and leaves to control a bacterial wilt disease caused by soil bacteria Ralstonia solanacearum, and/or bacterial soft rots.

Reference to Deposited Biological Material

The accession numbers of microorganisms that have been deposited in relation to the present invention are as follows.

(1) Bacillus sp. G4L1 strain (NITE BP-03204)
(2) Bacillus timonensis G5S1 strain (NITE BP-03206)
(3) Fictibacillus solisalsi G5L2 strain (NITE BP-03205)

## Claims

1. A Fictibacillus solisalsi G5L2 bacterial strain (NITE BP-03205).

2. A Bacillus sp. G4L1bacterial strain (NITE BP-03204).

3. A Bacillus timonensis GSS1 bacterial strain (NITE BP-03206).

4. A culture of the bacterial strain according to any one of claims 1 to 3, wherein the strain

   (i) is capable of controlling a plant disease by being applied to at least one of a plant seed, a plant root, a stem and leaf portion of a plant, a plant cultivation support, a nutrient solution, and soil; and/or
   (ii) has a high growth rate.

5. A plant disease control agent comprising one or more of the strains of claims 1 to 3 as an active component.

6. A plant disease control agent comprising a culture of one or more of the bacterial strains according to any one of claims 1 to 3, wherein the agent is capable of controlling

   (i) a plant disease by being applied to at least one of a plant seed, a plant root, a stem and leaf portion of a plant, a plant support, a nutrient solution, and soil;
   (ii) a disease of at least one of a vegetable, a fruit, an Oryza plant, and a Poaceae plant;
   (iii) a disease of a Solanaceae plant and/or a Brassicaceae plant; and/or
   (iv) a soil-borne plant disease by being applied to stems and leaves of a plant.

7. The agent according to claim 6, wherein the soil-borne plant disease is bacterial wilt of vegetables and/or bacterial soft rots of vegetables.

8. Use of the bacterial strain according to any one of claims 1 to 3, the culture according to claim 4 or the plant disease control agent according to claims 5 or 6 for controlling a plant disease.

9. A plant disease control method comprising contacting or mixing viable bacteria of one or more of the strains of claims 1 to 3 or the culture of claim 4 with a plant seed, a plant root, an aboveground part of a plant, a plant support, a nutrient solution, or soil.

10. The method according to claim 9, wherein the method controls a disease of at least one of a vegetable, a fruit, an Oryza plant, and a Poaceae plant.

11. The method according to claim 9 or 10, wherein the method controls a disease of a Solanaceae plant and/or a Brassicaceae plant.

**12.** The method according to any one of claims 9 to 11, wherein the method controls a soil-borne plant disease by being applied to stems and leaves of a plant.

**13.** The method according to any one of claims 9 to 12, wherein the method controls bacterial wilt of vegetables and/or bacterial soft rots of vegetables.


**Patentansprüche**

**1.** Bakterienstamm *Fictibacillus solisalsi* G5L2 (NITE BP-03205).

**2.** Bakterienstamm *Bacillus sp.* G4L1(NITE BP-03204).

**3.** Bakterienstamm *Bacillus timonensis* G5S1 (NITE BP-03206).

**4.** Kultur des Bakterienstamms nach einem der Ansprüche 1 bis 3, wobei der Stamm

(i) in der Lage ist, eine Pflanzenkrankheit durch Anwendung auf mindestens einem aus einem Pflanzensamen, einer Pflanzenwurzel, einem Stamm und einem Blattteil einer Pflanze, einer Pflanzenanbauunterlage, einer Nährlösung und Erde zu bekämpfen; und/oder
(ii) eine hohe Wachstumsrate aufweist.

**5.** Pflanzenkrankheitsbekämpfungsmittel, das einen oder mehrere Stämme nach den Ansprüchen 1 bis 3 als einen Wirkstoff umfasst.

**6.** Pflanzenkrankheitsbekämpfungsmittel, das eine Kultur eines oder mehrerer Bakterienstämme nach einem der Ansprüche 1 bis 3 umfasst, wobei das Mittel in der Lage ist,

(i) eine Pflanzenkrankheit durch Anwendung auf mindestens einem aus einem Pflanzensamen, einer Pflanzenwurzel, einem Stamm und einem Blattteil einer Pflanze, einer Pflanzenunterlage, einer Nährlösung und Erde zu bekämpfen;
(ii) eine Krankheit mindestens einer aus einem Gemüse, einer Frucht, einer Oryza-Pflanze und einer Poaceae-Pflanze zu bekämpfen,
(iii) eine Krankheit einer Solanaceae-Pflanze und/oder einer Brassicaceae-Pflanze zu bekämpfen; und/oder
(iv) eine bodenbürtige Pflanzenkrankheit durch Anwendung auf Stängel und Blätter einer Pflanze zu bekämpfen.

**7.** Mittel nach Anspruch 6, wobei die bodenbürtige Pflanzenkrankheit bakterielle Welke bei Gemüse und/oder bakterielle Weichfäule bei Gemüse ist.

**8.** Verwendung des Bakterienstamms nach einem der Ansprüche 1 bis 3, der Kultur nach Anspruch 4 oder des Pflanzenkrankheitsbekämpfungsmittels nach Anspruch 5 oder 6 zur Bekämpfung einer Pflanzenkrankheit.

**9.** Verfahren zur Bekämpfung von Pflanzenkrankheiten, umfassend das Inkontaktbringen oder Mischen von lebensfähigen Bakterien eines oder mehrerer der Stämme nach einem der Ansprüche 1 bis 3 oder der Kultur nach Anspruch 4 mit einem Pflanzensamen, einer Pflanzenwurzel, einem oberirdischen Teil einer Pflanze, einer Pflanzenunterlage, einer Nährlösung oder Erde.

**10.** Verfahren nach Anspruch 9, wobei das Verfahren eine Krankheit mindestens einer der folgenden bekämpft: eines Gemüse, einer Frucht, einer Oryza-Pflanze und einer Poaceae-Pflanze.

**11.** Verfahren nach Anspruch 9 oder 10, wobei das Verfahren eine Krankheit einer Solanaceae-Pflanze und/oder einer Brassicaceae-Pflanze bekämpft.

**12.** Verfahren nach einem der Ansprüche 9 bis 11, wobei das Verfahren eine bodenbürtige Pflanzenkrankheit durch Anwendung auf Stängel und Blätter einer Pflanze bekämpft.

**13.** Verfahren nach einem der Ansprüche 9 bis 12, wobei das Verfahren bakterielle Welke von Gemüse und/oder bakterielle Weichfäule von Gemüse bekämpft.

**Revendications**

1. Souche bactérienne G5L2 de Fictibacillus solisalsi (NITE BP-03205).

2. Souche bactérienne G4L1 de Bacillus sp. (NITE BP-03204).

3. Souche bactérienne G5S1 de Bacillus timonensis (NITE BP-03206).

4. Culture de la souche bactérienne selon l'une quelconque des revendications 1 à 3, dans laquelle la souche

   (i) est susceptible de contrôler une maladie végétale en étant appliquée sur au moins une graine de plante, une racine de plante, une partie tige et feuille d'une plante, un support de culture pour plante, une solution nutritive, et un sol ; et/ou
   (ii) a un taux de croissance élevé.

5. Agent de contrôle des maladies végétales comprenant une ou plusieurs des souches selon les revendications 1 à 3 en tant que composant actif.

6. Agent de contrôle des maladies végétales comprenant une culture d'une ou de plusieurs souches bactériennes selon l'une quelconque des revendications 1 à 3, où l'agent est susceptible de contrôler

   (i) une maladie végétale par l'application sur au moins une graine de plante, une racine de plante, une partie tige et feuille d'une plante, un support pour plante, une solution nutritive, et un sol ;
   (ii) une maladie d'au moins un légume, d'un fruit, d'une plante du genre Oryza, et d'une plante du genre Poacées ;
   (iii) une maladie d'une plante du genre Solanacées et/ou d'une plante du genre Brassicacées ; et/ou
   (iv) une maladie végétale transmise par le sol par l'application sur des tiges et des feuilles d'une plante.

7. Agent selon la revendication 6, dans lequel la maladie végétale transmise par le sol est une flétrissure bactérienne des légumes et/ou des pourritures molles bactériennes des légumes.

8. Utilisation de la souche bactérienne selon l'une quelconque des revendications 1 à 3, de la culture selon la revendication 4 ou de l'agent de contrôle des maladies végétales selon les revendications 5 ou 6 pour le contrôle d'une maladie végétale.

9. Méthode de contrôle des maladies végétales comprenant la mise en contact ou le mélange de bactéries viables d'une ou de plusieurs des souches selon les revendications 1 à 3 ou la culture selon la revendication 4 avec une graine de plante, une racine de plante, une partie souterraine d'une plante, un support pour plante, une solution nutritive, ou un sol.

10. Méthode selon la revendication 9, où la méthode contrôle une maladie d'au moins un légume, un fruit, une plante du genre Oryza et d'une plante du genre Poacées.

11. Méthode selon la revendication 9 ou 10, où la méthode contrôle une maladie d'une plante du genre Solanacées et/ou d'une plante du genre Brassicacées.

12. Méthode selon l'une quelconque des revendications 9 à 11, où la méthode contrôle une maladie d'une plante transmise par le sol par l'application sur les tiges et les feuilles d'une plante.

13. Méthode selon l'une quelconque des revendications 9 à 12, où la méthode contrôle une flétrissure bactérienne des légumes et/ou des pourritures molles bactériennes des légumes.

[FIG. 1]

[FIG. 2]

[FIG. 3]

ALUMINUM FOIL

[FIG. 4]

GROWTH CURVES OF FOUR BACTERIAL STRAINS

····◆····IMPRESSION CLEAR     ····●···· BOTOKILLER WETTABLE POWDER     ····▲····ECOSHOT     ━■━ G4L1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2012211124 A
- WO 2018228986 A
- WO 2020114934 A
- WO 2014064094 A
- WO 2015162164 A
- WO 2017178582 A
- WO 2018015476 A
- WO 2015059262 A

### Non-patent literature cited in the description

- *Biol. control*, 2016, vol. 100, 63-69
- *FEMSMicrobiol. Ecol*, 2008, vol. 65, 565-573
- **KAGAKU ; SEIBUTSU**. *Chemistry and Biology*, 2013, vol. 51 (8), 541-547
- *Proc. Natl. Acad. Sci. USA*, 10 November 2009, vol. 106 (45), 19126-19131
- *Stand Genomic Sci*, 28 February 2010, vol. 2 (1), 117-134
- *Int J Syst Evol Microbiol*, 01 January 2007, vol. 57 (1), 81-91
- *CHEMICAL ABSTRACTS*, 1231214-23-5
- *CHEMICAL ABSTRACTS*, 1472052-11-1
- *CHEMICAL ABSTRACTS*, 1472050-34-2
- *CHEMICAL ABSTRACTS*, 1448758-62-0
- *CHEMICAL ABSTRACTS*, 1448761-28-1
- *CHEMICAL ABSTRACTS*, 1472047-71-4
- *CHEMICAL ABSTRACTS*, 2266183-40-6
- *CHEMICAL ABSTRACTS*, 1606999-43-2
- *CHEMICAL ABSTRACTS*, 2266190-06-9
- *CHEMICAL ABSTRACTS*, 2266164-36-5
- *CHEMICAL ABSTRACTS*, 2266170-31-2
- *CHEMICAL ABSTRACTS*, 2138855-12-4
- *CHEMICAL ABSTRACTS*, 1708087-22-2
- *CHEMICAL ABSTRACTS*, 1820807-75-7
- *CHEMICAL ABSTRACTS*, 1606999-21-6
- *CHEMICAL ABSTRACTS*, 2266292-43-5
- *CHEMICAL ABSTRACTS*, 1607001-97-7
- *CHEMICAL ABSTRACTS*, 1083-55-2